# EUROPEAN PATENT APPLICATION

(11) **EP 4 840 854 A1**
(43) Date of publication of application: **18.11.2026**
(21) Application number: 24916675.2
(22) Date of filing: 16.12.2024
(51) Int. Cl.: C07D 401/06, A61K 31/454, A61P 27/00

(54) **PIPERIDINYL INDOLE DERIVATIVE, PREPARATION METHOD THEREFOR AND USE THEREOF**

(30) Priority: 10.01.2024 CN 202410033993
(71) Applicant: Hebei Yiling Medicine Research Institute Co., Ltd, Hebei 050035 (CN)
(72) Inventor: JIA, Zhenhua, Shijiazhuang, Hebei 050035 (CN); HOU, Yunlong, Shijiazhuang, Hebei 050035 (CN); LI, Mengnan, Shijiazhuang, Hebei 050035 (CN); GUO, Zhifang, Shijiazhuang, Hebei 050035 (CN); TONG, Yanan, Shijiazhuang, Hebei 050035 (CN); LI, Faguang, Shijiazhuang, Hebei 050035 (CN); WANG, Xiaohang, Shijiazhuang, Hebei 050035 (CN)
(74) Representative: Bird & Bird LLP
(86) International application number: PCT/CN2024/139628
(87) International publication number: WO 2025/148628

(57) **Abstract**

The present invention provides a piperidinyl indole derivative, a preparation method therefor and a use thereof. Specifically, the present invention provides compounds having structural formulas A and I, or pharmaceutically acceptable forms thereof, which can be used for preparing a medicament for treating diseases or disorders mediated by alternative complement pathway dysfunction, and particularly for preparing a medicament for treating diseases or disorders mediated by complement factor B.

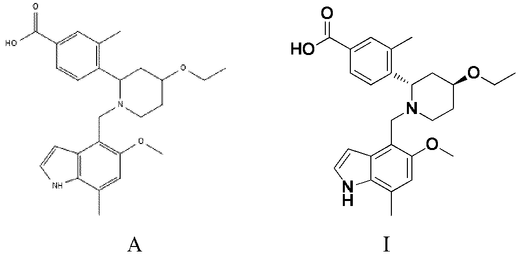

## Description

### TECHNICAL FIELD

The present invention relates to piperidinyl indole derivatives and uses thereof, and in particular, to piperidinyl indole derivatives, methods for preparing the same, and their use in the treatment of diseases or disorders associated with activation of the complement alternative pathway, especially diseases or disorders related to complement factor B.

### BACKGROUND

The complement system is mainly composed of various proteins synthesized by the liver. These proteins circulate in blood and tissues and account for approximately 10% of serum globulins. The complement system is an important component of the innate immune system. In normal host cells, complement activation is tightly regulated by a variety of cell-surface regulatory proteins, , which prevents damage to host tissues. In contrast, foreign pathogens and damaged or altered host cells are vulnerable to such attack. Abnormal or excessive activation of the complement system may cause cellular and tissue damage, leading to autoimmune and inflammatory diseases.

The complement system consists mainly of complement activation components, regulatory factors, and complement receptors. Under physiological conditions, complement activation components exist as inactive zymogens. After the complement system is activated, a membrane attack complex is formed, which creates pores in the cell membrane and ultimately causes death of the target cell. Complement regulatory factors precisely regulate various steps of complement activation. Complement receptors, which are primarily expressed on the surface of immune cells, are a class of membrane proteins. By binding to activated complement components, complement receptors mediate functions such as recruiting leukocytes to sites of inflammation, promoting phagocytosis of pathogenic microorganisms, and clearing immune complexes from the blood.

The complement system is primarily activated via three pathways: the classical pathway (CP), the lectin pathway (LP), and the alternative pathway (AP).

The classical pathway and the lectin pathway are triggered by recognition of signals on the cell surface. In both pathways, C4 and C2 molecules are cleaved to form C4b2a. In the classical pathway, this reaction is mediated by the C1 complex. In the lectin pathway, certain pathogen-associated molecular patterns (PAMPs) activate mannose-binding lectin-associated serine protease zymogens (MASPs), and MASP-1 and MASP-2 further form complexes that promote cleavage of C4 and C2, ultimately generating the C3 convertase C4b2a.

The alternative pathway differs from the above two pathways. It is initiated by hydrolysis of C3, which exposes the thioester domain. In the presence of complement factor B (CFB) and complement factor D (CFD), hydrolyzed C3 binds to activated Bb to form the active C3 convertase (C3(H₂O)Bb). C3 molecules are then cleaved by C3 convertase into C3a and C3b. Surface-deposited C3b can form a new C3 convertase, C3bBb, in the presence of CFB, thereby establishing a C3 reaction cycle.

In addition, excess C3b molecules may bind to C3 convertases to form C5 convertases, including C4b2aC3b and C3bBbC3b, which cleave C5 into C5a and C5b. C5b subsequently binds to C6, C7, C8, and C9 to form the membrane attack complex (C5b9), ultimately causing damage to target cells.

Current studies demonstrate that various hematologic, autoimmune, inflammatory, and neurodegenerative diseases are associated with dysregulation of the complement system. Examples include paroxysmal nocturnal hemoglobinuria (PNH), IgA nephropathy (IgAN), atypical hemolytic uremic syndrome (aHUS), C3 glomerulopathy (C3G), age-related macular degeneration (AMD), systemic lupus erythematosus (SLE), dense deposit disease (DDD), ANCA-associated vasculitis (AAV), immune thrombocytopenia (ITP), and cold agglutinin disease (CAD).

Paroxysmal nocturnal hemoglobinuria (PNH) is a rare acquired clonal disorder of hematopoietic stem cells. Its clinical manifestations include paroxysmal intravascular hemolysis of varying severity, paroxysmal hemoglobinuria, bone marrow failure, and venous thrombosis. Deficiency of the complement decay-accelerating factor CD55 and the membrane attack complex inhibitor CD59 is considered the primary mechanism underlying intravascular hemolysis in patients with PNH. CD55 and CD59 are physiologically expressed on the cell surface. CD55 inhibits complement activation by accelerating the decay of C3 and C5 convertases, whereas CD59 directly inhibits formation of the membrane attack complex (MAC) by preventing insertion of C9 into the C5b-8 complex during MAC assembly, thereby suppressing the terminal complement attack response.

IgA nephropathy is a primary glomerular disease characterized by predominant deposition of IgA in the glomerular mesangial region, with or without deposition of other immunoglobulins in the glomerular mesangial region.

IgA nephropathy is caused by multiple factors. The internationally accepted pathogenesis involves an increase in galactose-deficient IgA1 (Gd-IgA1) caused by genetic or environmental factors; production of specific antibodies against Gd-IgA1; formation of IgA-containing pathogenic immune complexes through binding of Gd-IgA1 to such antibodies; and glomerular injury caused by these immune complexes. Complement activation is one of the most common downstream events following deposition of Gd-IgA1 immune complexes in the renal mesangial region of patients with IgAN, suggesting that abnormal activation of the complement alternative pathway is associated with the severity of clinical and pathological lesions.

C3 glomerulopathy (C3G) refers to a group of diseases characterized by abnormal deposition of complement C3 in the glomeruli due to hereditary or acquired defects in regulation of the complement alternative pathway. C3G may manifest as asymptomatic hematuria and/or proteinuria, acute nephritic syndrome, nephrotic syndrome, rapidly progressive nephritic syndrome, and other manifestations. Dysregulation of the complement alternative pathway is a major driver of the pathogenesis of C3G.

Atypical hemolytic uremic syndrome (aHUS) is a rare and life-threatening clinical syndrome characterized by microangiopathic hemolytic anemia, thrombocytopenia, and organ injury, particularly acute kidney injury. Persistent activation of the alternative complement pathway, caused by congenital abnormalities in alternative pathway proteins or by autoantibodies targeting complement factors or complement pathway regulatory proteins, is the major pathogenic mechanism of aHUS.

Age-related macular degeneration (AMD) is a multifactorial degenerative disease and a leading cause of visual impairment and blindness in older adults. The global number of individuals affected by AMD is expected to reach 288 million by 2040. Large-scale studies have demonstrated that complement activation plays an important role in the pathogenesis of AMD. Inhibition of the complement system, which involves inhibiting complement proteins to downregulate complement pathway activation and prevent formation of the membrane attack complex, is a potential therapeutic strategy for dAMD.

ANCA-associated vasculitis (AAV) is a group of systemic diseases characterized by immune necrotizing inflammation of small blood vessels. AAV is characterized by the presence of antibodies against neutrophil proteinase 3 (PR3-ANCA) or myeloperoxidase (MPO-ANCA). These antibodies can activate neutrophils and the complement system, resulting in vascular inflammation and injury. The interaction between the complement activation product C5a and its receptor C5aR1 (CD88) is widely recognized as a key step in the initiation of inflammatory responses and has become an important target for therapeutic intervention.

Systemic lupus erythematosus (SLE) is a chronic systemic autoimmune disease involving multiple organs. It is characterized by the production of various pathogenic autoantibodies and widespread deposition of immune complexes (IC). Approximately 50% to 60% of patients with SLE develop lupus nephritis (LN) within 10 years of disease onset, and LN is one of the major contributors to poor prognosis in patients with SLE. Studies have shown that the formation and deposition of large amounts of immune complexes, together with excessive complement activation or dysregulation of the complement system, are closely associated with the onset and progression of LN. Complement activation is an important marker of disease activity and relapse in LN.

Immune thrombocytopenia (ITP) is a disorder characterized by reduced platelet counts resulting from increased platelet destruction and impaired platelet production. Substantial experimental and clinical evidence indicates that the production of autoantibodies capable of accelerating platelet clearance plays a central role in the pathogenesis of ITP. Such IgM and IgG anti-platelet antibodies can activate complement in vivo, thereby amplifying the complement cascade and aggravating disease progression. Early studies have shown that inhibition of terminal complement activation at C5 may increase platelet counts and rapidly reduce thromboembolic complications.

Cold agglutinin disease (CAD) is an autoimmune hemolytic anemia (AIHA) that is affected by low temperatures and caused by cold agglutinins (CAs). In this disease, the complement system, a component of the immune system, mistakenly targets healthy red blood cells, causing their destruction (hemolysis). Patients with CAD may experience chronic anemia, severe fatigue, acute hemolytic crises, and other complications, including an increased risk of thromboembolic events and premature death. Current treatment options for CAD include both non-pharmacological and pharmacological approaches. Pharmacological treatments include general supportive treatment, B-cell-directed therapy, and complement inhibitor therapy. Complement inhibitors have a rapid onset of action and may be used for the treatment of acute hemolysis. However, long-term maintenance therapy is generally required to prevent hemolysis.

### SUMMARY

An objective of the present invention is to provide a novel compound. The compound can be used to modulate activation of the alternative complement pathway.

Another objective of the present invention is to provide a method for preparing the compound.

A further objective of the present invention is to provide a composition comprising the compound.

Still another objective of the present invention is to provide a use of the compound.

### First Technical Solution

The present invention provides a compound of Formula A, or a pharmaceutically acceptable form thereof:

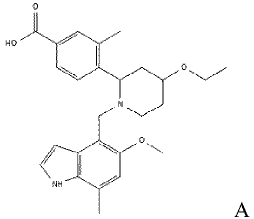

Preferably, the compound is a stereoisomer represented by Formula I:

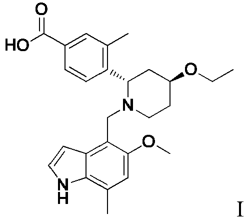

According to some specific embodiments of the present invention, the pharmaceutically acceptable form of the compound of Formula A or Formula I is selected from the group consisting of a pharmaceutically acceptable salt, a deuterated derivative, a prodrug, a polymorph, and a solvate.

The pharmaceutically acceptable salt generally refers to any salt that is physiologically tolerable when used appropriately for therapeutic purposes, particularly when administered to or used in humans and/or mammals (generally, this means that it is non-toxic, particularly with respect to its counterion). Such physiologically acceptable salts may be formed from the compound with a suitable cation or base, or from the compound with a suitable anion or acid. In particular, the salts may include salts formed with alkali metal cations, alkaline earth metal cations, or an ammonium cations (NH₄⁺), as well as acid addition salts formed with hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid, or citric acid. Exemplary chemical structures of pharmaceutically acceptable salts of the compound of Formula I are shown below:

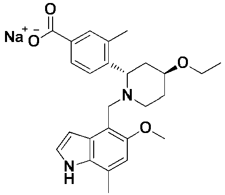

Deuterated derivative refers to a compound obtained by replacing any hydrogen atom in the compound of Formula I with deuterium. Such a compound may provide certain therapeutic advantages due to enhanced metabolic stability, such as an increased in vivo half-life, reduced dosage requirements, or an improved therapeutic index.

According to some specific embodiments of the present invention, the deuterated derivative is selected from the following structures:

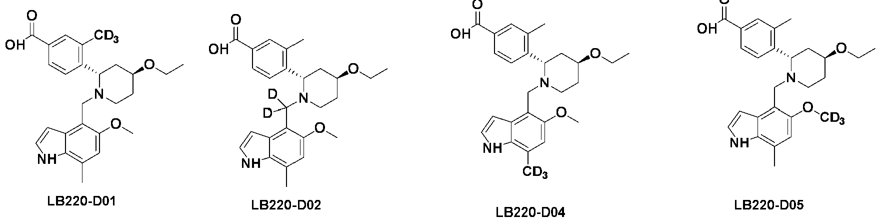

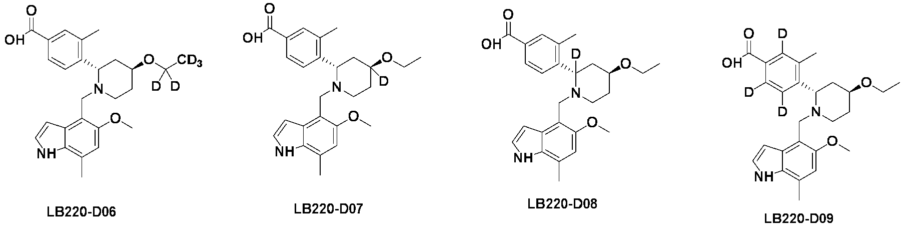

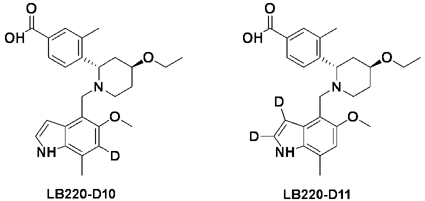

The prodrug refers to a derivative that can be converted into the compound of Formula I in vivo, thereby improving the bioavailability or delivery efficiency of the compound.

The polymorph refers to any crystalline form of the compound, which may exist either as a single crystalline form or as a combination of multiple crystalline forms.

The solvate generally refers to a substance obtained by associating an active compound according to the present invention, in any form, with another molecule, usually a polar solvent, through non-covalent interactions. The resulting substance specifically includes, but is not limited to, a hydrate or an alcohol solvate, such as a methanol solvate.

### Technical Solution II:

The present invention provides a method for preparing the compound of Formula A, comprising the following steps:
(1) reacting 4-bromo-3-methylbenzonitrile, 4-methoxypyridine, and benzyl chloroformate under catalyzation action a Grignard reagent to produce benzyl 2-(4-cyano-2-methylphenyl)-4-oxo-3,4-dihydropyridine-1(2H)-carboxylate;
(2) subjecting benzyl 2-(4-cyano-2-methylphenyl)-4-oxo-3,4-dihydropyridine-1(2H)-carboxylate to hydrogenation to obtain benzyl 2-(4-cyano-2-methylphenyl)-4-oxopiperidine-1-carboxylate;
(3) reducing the carbonyl group of benzyl 2-(4-cyano-2-methylphenyl)-4-oxopiperidine-1-carboxylate to obtain benzyl 2-(4-cyano-2-methylphenyl)-4-hydroxypiperidine-1-carboxylate;
(4) reacting benzyl 2-(4-cyano-2-methylphenyl)-4-hydroxypiperidine-1-carboxylate with a silylating reagent in the presence of an acid-binding agent to protect the hydroxy group, thereby obtaining 4-((tert-butyldiphenylsilyl)oxy)-2-(4-cyano-2-methylphenyl)piperidine-1-carboxylate;
(5) removing the hydroxy-protecting group from 4-((tert-butyldiphenylsilyl)oxy)-2-(4-cyano-2-methylphenyl)piperidine-1-carboxylate to obtain 2-(4-cyano-2-methylphenyl)-4-hydroxypiperidine-1-carboxylate;
(6) subjecting 2-(4-cyano-2-methylphenyl)-4-hydroxypiperidine-1-carboxylate to a nucleophilic substitution reaction with a halohydrocarbon to obtain 2-(4-cyano-2-methylphenyl)-4-ethoxypiperidine-1-carboxylate;
(7) reacting 2-(4-cyano-2-methylphenyl)-4-ethoxypiperidine-1-carboxylate with an acid and an alkyl alcohol to obtain an alkyl 4-(4-ethoxypiperidin-2-yl)-3-methylbenzoate;
(8) reacting the alkyl 4-(4-ethoxypiperidin-2-yl)-3-methylbenzoate with tert-butyl 4-formyl-5-methoxy-7-methyl-1H-indole-1-carboxylate to obtain tert-butyl 4-((4-ethoxy-2-(4-(alkoxycarbonyl)-2-methylphenyl)piperidin-1-yl)methyl)-5-methoxy-7-methyl-1H-indole-1-carboxylate;
(9) hydrolyzing tert-butyl 4-((4-ethoxy-2-(4-(methoxycarbonyl)-2-methylphenyl)piperidin-1-yl)methyl)-5-methoxy-7-methyl-1H-indole-1-carboxylate to obtain the compound of Formula A, namely 4-(4-ethoxy-1-((5-methoxy-7-methyl-1H-indol-4-yl)methyl)piperidin-2-yl)-3-methylbenzoic acid.

The compound of Formula I may be obtained by subjecting the compound of Formula A to SFC separation. The specific preparation method is as follows:
(1) reacting 4-bromo-3-methylbenzonitrile, 4-methoxypyridine, and benzyl chloroformate under catalyzation action of a Grignard reagent to produce benzyl 2-(4-cyano-2-methylphenyl)-4-oxo-3,4-dihydropyridine-1(2H)-carboxylate;
(2) subjecting benzyl 2-(4-cyano-2-methylphenyl)-4-oxo-3,4-dihydropyridine-1(2H)-carboxylate to hydrogenation to obtain benzyl 2-(4-cyano-2-methylphenyl)-4-oxopiperidine-1-carboxylate;
(3) reducing the carbonyl group of benzyl 2-(4-cyano-2-methylphenyl)-4-oxopiperidine-1-carboxylate to obtain benzyl 2-(4-cyano-2-methylphenyl)-4-hydroxypiperidine-1-carboxylate;
(4) reacting benzyl 2-(4-cyano-2-methylphenyl)-4-hydroxypiperidine-1-carboxylate with a silylating reagent in the presence of an acid-binding agent to protect the hydroxy group, followed by purification, to obtain trans-4-((tert-butyldiphenylsilyl)oxy)-2-(4-cyano-2-methylphenyl)piperidine-1-carboxylate;
(5) removing the hydroxy-protecting group from trans-4-((tert-butyldiphenylsilyl)oxy)-2-(4-cyano-2-methylphenyl)piperidine-1-carboxylate to obtain trans-2-(4-cyano-2-methylphenyl)-4-hydroxypiperidine-1-carboxylate;
(6) subjecting trans-2-(4-cyano-2-methylphenyl)-4-hydroxypiperidine-1-carboxylate to a nucleophilic substitution reaction with a halohydrocarbon to obtain trans-2-(4-cyano-2-methylphenyl)-4-ethoxypiperidine-1-carboxylate;
(7) reacting trans-2-(4-cyano-2-methylphenyl)-4-ethoxypiperidine-1-carboxylate with an acid and an alkyl alcohol to obtain a trans-alkyl 4-(4-ethoxypiperidin-2-yl)-3-methylbenzoate;
(8) reacting the trans- 4-(4-ethoxypiperidin-2-yl)-3-methylbenzoate with tert-butyl 4-formyl-5-methoxy-7-methyl-1H-indole-1-carboxylate to obtain tert-butyl trans-4-(4-trans-ethoxy-2-(4-(alkoxycarbonyl)-2-methylphenyl)piperidin-1-yl)methyl)-5-methoxy-7-methyl-1H-indole-1-carboxylate;
(9) subjecting tert-butyl trans-4-(4-transethoxy-2-(4-(alkoxycarbonyl)-2-methylphenyl)piperidin-1-yl)methyl)-5-methoxy-7-methyl-1H-indole-1-carboxylate to SFC purification to obtain tert-butyl 4-((2S,4S)-4-ethoxy-2-(4-(alkoxycarbonyl)-2-methylphenyl)piperidin-1-yl)methyl)-5-methoxy-7-methyl-1H-indole-1-carboxylate;
(10) hydrolyzing tert-butyl 4-((2S,4S)-4-ethoxy-2-(4-(alkoxycarbonyl)-2-methylphenyl)piperidin-1-yl)methyl)-5-methoxy-7-methyl-1H-indole-1-carboxylate to obtain the compound of Formula I, namely 4-((2S,4S)-4-ethoxy-1-((5-methoxy-7-methyl-1H-indol-4-yl)methyl)piperidin-2-yl)-3-methylbenzoic acid.

In a specific embodiment of the above preparation method, the Grignard reagent used in step (1) is isopropylmagnesium bromide or isopropylmagnesium chloride.

In a specific embodiment of the above preparation method, the silylating reagent used in step (4) is tert-butyldiphenylsilyl chloride, and the acid scavenger is imidazole.

In a specific embodiment of the above preparation method, the alkyl halide used in step (6) is ethyl iodide.

In a specific embodiment of the above preparation method, the acid used in step (7) is sulfuric acid, and the alkyl alcohol is methanol.

### Technical Solution III:

The present invention further provides a composition comprising a compound of Formula I, or a pharmaceutically acceptable form thereof, and a pharmaceutically acceptable carrier or excipient, and/or one or more additional therapeutic agents.

Carrier refers to a material capable of modifying the manner in which a drug enters the human body, controlling the release rate of the drug, and delivering the drug to a target organ. The carrier causes no significant irritation to the human body and does not substantially impair the biological activity of the drug. Excipient refers to a material that, together with a drug, forms a particular dosage form to facilitate administration. The excipient is compatible with the drug, does not cause adverse effects, and does not impair the therapeutic efficacy of the drug. Additional therapeutic agent refers to any other drug that can be co-administered with the compound of Formula I without causing an adverse interaction therewith.

### Technical Solution IV:

The present invention further provides the use of a compound of Formula A or Formula I, a pharmaceutically acceptable form thereof, or a pharmaceutical composition comprising the same, in the manufacture of a medicament for treating a disease or disorder mediated by dysregulation of the alternative complement pathway.

In some embodiments of the present invention, the disease or disorder mediated by dysregulation of the alternative complement pathway is mediated by complement factor B.

In some embodiments of the present invention, the disease or disorder is a hematological, autoimmune, inflammatory, and/or neurodegenerative disease or disorder.

In some embodiments of the present invention, the disease or disorder is selected from diseases or disorders mediated by dysregulation of the alternative complement pathway, such as paroxysmal nocturnal hemoglobinuria, immunoglobulin A nephropathy, atypical hemolytic uremic syndrome, age-related macular degeneration, ANCA-associated vasculitis, systemic lupus erythematosus, immune thrombocytopenia, cold agglutinin disease, and/or C3 glomerulopathy.

### DESCRIPTION OF EMBODIMENTS

The technical solutions of the present invention are described in detail below with reference to specific examples. Unless otherwise indicated, examples for which no specific conditions are provided were carried out under conventional conditions or under conditions recommended by the manufacturers. Unless otherwise indicated, reagents and apparatus for which no manufacturer is specified are commercially available. Unless otherwise specified, column chromatography was performed using 200-300 mesh silica gel, and room temperature refers to 20-30°C.

### Abbreviations

CbzCl: benzyl chloroformate
EA: ethyl acetate
PE: petroleum ether
MTBE: methyl tert-butyl ether
TBDPSCl: tert-butyldiphenylsilyl chloride
SFC: supercritical fluid chromatography

### Example 1

This example provides the compound of Formula I. The compound was prepared according to the following:

### Synthetic Route:

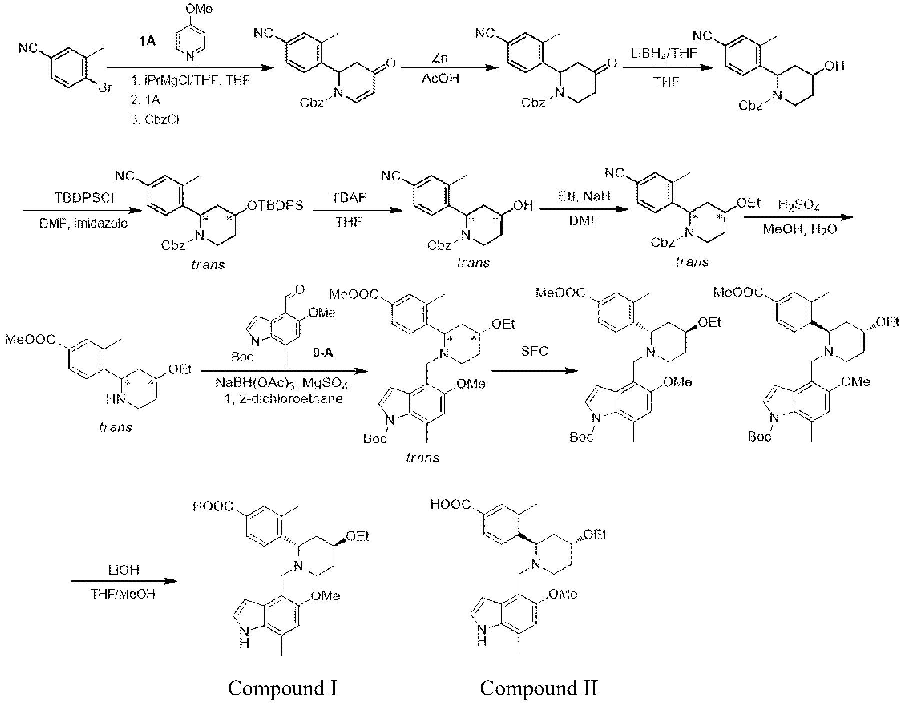

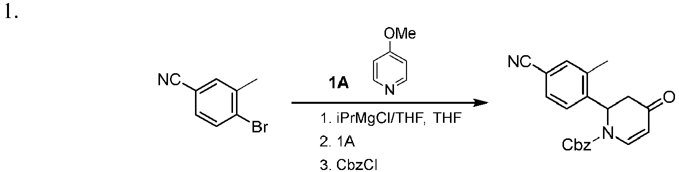

Isopropylmagnesium chloride in tetrahydrofuran (THF, 1.3 M, 112 mL) was added dropwise to a solution of 4-bromo-3-methylbenzonitrile (27 g, 138 mmol) in tetrahydrofuran (108 mL) at 10-15°C. After the addition was complete, additional tetrahydrofuran (108 mL) was added, and the reaction mixture was cooled to -5°C. 4-Methoxypyridine (15 g, 138 mmol) and CbzCl (23.5 g, 138 mmol) were added, and the resulting mixture was stirred at -5°C for 10 h. After completion of the reaction, the reaction was quenched with HCl (5 M, 60 mL) and extracted with EA (200 mL). The organic phase was washed with saturated aqueous sodium chloride (100 mL), dried over anhydrous sodium sulfate, and then the solvent was removed. The crude product was purified by silica gel column chromatography using EA/PE(1/2), to afford benzyl 2-(4-cyano-2-methylphenyl)-4-oxo-3,4-dihydropyridine-1(2H)-carboxylate (28.8 g, yield 60.4%).

LCMS (ESI, m/z): [M+H] ⁺ = 347.1.

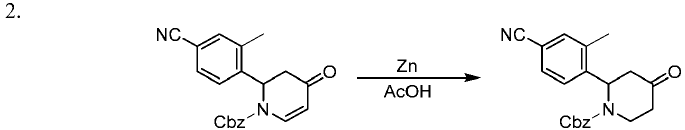

Under a nitrogen atmosphere, zinc powder (13.2 g, 202 mmol) was added at room temperature to a solution of benzyl 2-(4-cyano-2-methylphenyl)-4-oxo-3,4-dihydropyridine-1(2H)-carboxylate (28.0 g, 80.8 mmol) in acetic acid (100 mL). The reaction was carried out at 80°C for 1 h. After the reaction was complete, the reaction mixture was filtered to remove the filter cake. The filtrate was concentrated to dryness by rotary evaporation. The crude product was dissolved in MTBE (150 mL) and water (80 mL). The layers were separated, and the organic phase was collected, washed with saturated sodium chloride solution (80 mL), and dried over anhydrous sodium sulfate. The mixture was filtered, and concentrated under reduced pressure to give benzyl 2-(4-cyano-2-methylphenyl)-4-oxopiperidine-1-carboxylate (18.2 g).

LCMS (ESI, m/z): [M+H] ⁺ = 349.0.

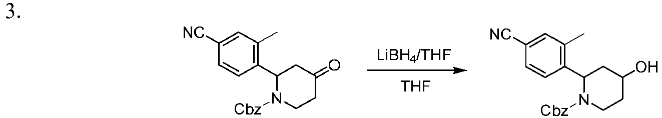

Under a nitrogen atmosphere, a solution of lithium borohydride in tetrahydrofuran (2M, 36.9 mL) was added dropwise at -45°C to a solution of benzyl 2-(4-cyano-2-methylphenyl)-4-oxopiperidine-1-carboxylate (19.5 g, 55.9 mmol) in tetrahydrofuran (195 mL). The reaction was carried out at -45°C for 1 h. After completion of the reaction, the reaction mixture was quenched by adding saturated aqueous potassium bisulfate solution (100 mL). MTBE (300 mL) and brine (300 mL) were added, and layered. The organic phase was dried over anhydrous sodium sulfate and then concentrated to dryness by rotary evaporation. Benzyl 2-(4-cyano-2-methylphenyl)-4-hydroxypiperidine-1-carboxylate (19.0 g, yield: 96.8%) was afforded.

LCMS (ESI, m/z): [M+H] ⁺ = 351.0.

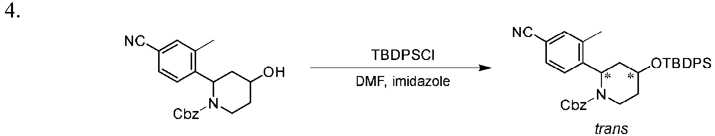

Benzyl 2-(4-cyano-2-methylphenyl)-4-hydroxypiperidine-1-carboxylate (19 g, 54.2 mmol, 1.0 eq) was dissolved in DMF (190 mL). TBDPSCl (19.4 g, 70.5 mmol, 1.3 eq) and imidazole (5.02 g, 73.7 mmol, 1.36 eq) were added at room temperature, and the resulting mixture was stirred at room temperature for 12 h. After completion of the reaction, saturated brine (150 mL) and MTBE (200 mL) were added. The layers were separated, and the organic phase was washed with saturated brine (50 mL), dried over anhydrous sodium sulfate, filtered, and concentrated under reduced pressure. The residue was purified by column chromatography (EA/PE=1/10) to afford benzyl trans-4-((tert-butyldiphenylsilyl)oxy)-2-(4-cyano-2-methylphenyl)piperidine-1-carboxylate (4 g, yield 12.5%).

LCMS (ESI, m/z): [M+H] ⁺ = 589.2.

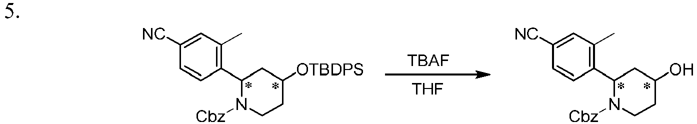

Benzyl trans-4-((tert-butyldiphenylsilyl)oxy)-2-(4-cyano-2-methylphenyl)piperidine-1-carboxylate (3 g, 5.1 mmol, 1.0 eq) was dissolved in tetrahydrofuran (30 mL). Tetrabutylammonium fluoride (2.41 g, 7.64 mmol) was added at room temperature, and the resulting mixture was stirred at room temperature for 1 h. After completion of the reaction, saturated brine (20 mL) and MTBE (10 mL) were added. The mixture was stirred, and layered. The organic phase was dried over anhydrous sodium sulfate, filtered, and concentrated under reduced pressure. The crude product was purified by column chromatography (EA/PE=1/10-1/2), to afford trans-2-(4-cyano-2-methylphenyl)-4-hydroxypiperidine-1-carboxylate (1.75 g, yield 98.02%).

LCMS (ESI, m/z): [M+H] ⁺ = 351.1.

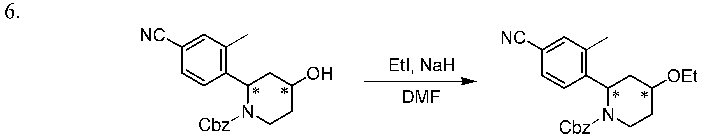

Sodium hydride (499 mg, 12.5 mmol, 60% dispersion in mineral oil) was suspended in DMF (9 mL), and the suspension was cooled to -5°C. trans-2-(4-cyano-2-methylphenyl)-4-hydroxypiperidine-1-carboxylate (1.75 g, 4.99 mmol) was added to the reaction solution, and then the reaction mixture was stirred at -5 to 0°C for 30 min. Iodoethane (2.65 g, 17 mmol) was added dropwise, and the resulting mixture was stirred at -5 to 0°C for 1 h. Upon completion, the reaction mixture was quenched by the addition of saturated potassium bisulfate solution (30 mL). The mixture was extracted with ethyl acetate (80 mL), and the organic phase was dried over anhydrous sodium sulfate, filtered, and concentrated under reduced pressure. The crude product was purified by column chromatography (EA/PE=1/5) to afford benzyl trans-2-(4-cyano-2-methylphenyl)-4-ethoxypiperidine-1-carboxylate (1.3 g, yield 68.8%).

LCMS (ESI, m/z): [M+H] ⁺ = 379.1.

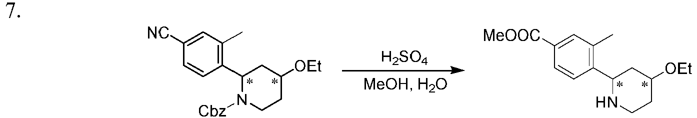

Trans-2-(4-cyano-2-methylphenyl)-4-ethoxypiperidine-1-carboxylate (1.3 g, 3.43 mmol) was dissolved in methanol (13 mL) and water (13 mL). Sulfuric acid (23.9 g, 243 mmol) was added, and the reaction was carried out at 80 °C for 12 h. After completion of the reaction, the mixture was neutralized to pH 8-9 with saturated sodium carbonate solution. The mixture was extracted with ethyl acetate (40 mL), and the organic phase was dried over anhydrous sodium sulfate, filtered, and concentrated under reduced pressure. The crude product was purified by column chromatography (DCM/MeOH=5/1) to afford methyl trans-4-(4-ethoxypiperidin-2-yl)-3-methylbenzoate (600 mg, yield 62.9%).

LCMS (ESI, m/z): [M+H] ⁺ = 278.0.

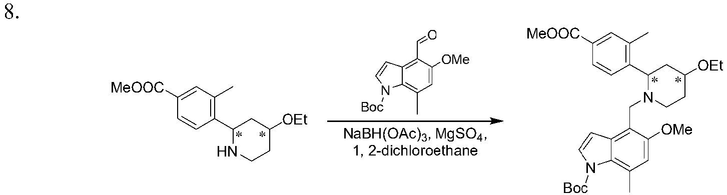

Under a nitrogen atmosphere, magnesium sulfate (796 mg, 6.62 mmol) and tert-butyl 4-formyl-5-methoxy-7-methyl-1H-indole-1-carboxylate (1.26 g, 4.37 mmol) were added to a solution of methyl trans-4-(4-ethoxypiperidin-2-yl)-3-methylbenzoate (600 mg, 2.16 mmol) in 1,2-dichloroethane (12 mL) at room temperature. The reaction was carried out for 4 h at room temperature. Sodium triacetoxyborohydride (1.63 g, 7.68 mmol) was then added to the reaction solution, and the reaction was carried out for 12 h. After the completion of the reaction, the reaction mixture was filtered, and the filtrate was concentrated under reduced pressure to give a crude product. The crude product was purified by preparative liquid chromatography to afford tert-butyl trans-4-(4-trans-ethoxy-2-(4-(methoxycarbonyl)-2-methylphenyl)piperidin-1-yl)methyl)-5-methoxy-7-methyl-1H-indole-1-carboxylate (500 mg, yield 41.9%). The conditions are as follows (column: Welch Ultimate XB-CN, 250 × 50, 10 µm; mobile phase: n-hexane/ethanol at a volume ratio of 97:3, isocratic elution; flow rate: 60 mL/min; wavelength: 254/220 nm; RT = 8.5 min.

LCMS (ESI, m/z): [M+H] ⁺ = 551.3.

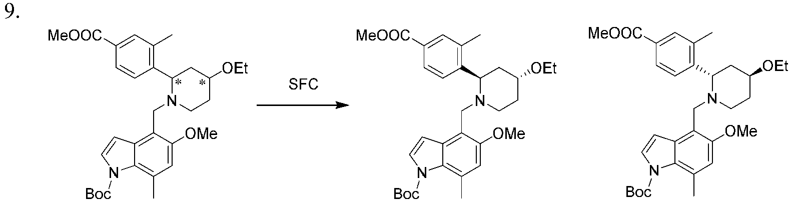

tert-Butyl trans-4-ethoxy-2-(4-(methoxycarbonyl)-2-methylphenyl)piperidin-1-yl)methyl)-5-methoxy-7-methyl-1H-indole-1-carboxylate (500 mg) was purified by SFC to afford tert-butyl 4-((2R,4R)-4-ethoxy-2-(4-(methoxycarbonyl)-2-methylphenyl)piperidin-1-yl)methyl)-5-methoxy-7-methyl-1H-indole-1-carboxylate (210 mg, yield 42%) and tert-butyl 4-((2S,4S)-4-ethoxy-2-(4-(methoxycarbonyl)-2-methylphenyl)piperidin-1-yl)methyl)-5-methoxy-7-methyl-1H-indole-1-carboxylate (165 mg, yield 33%). SFC conditions: column: DAICEL CHIRALPAK AD, 250 mm × 30 mm, 10 µm; mobile phase: CO₂/MeOH = 7:3, isocratic elution; wavelength: 254/220 nm; flow rate: 40 mL/min. The retention times of the two products were RT1 = 10.2 min, RT2 = 16.5 min, respectively.

LCMS (ESI, m/z): [M+H] ⁺ = 551.3.

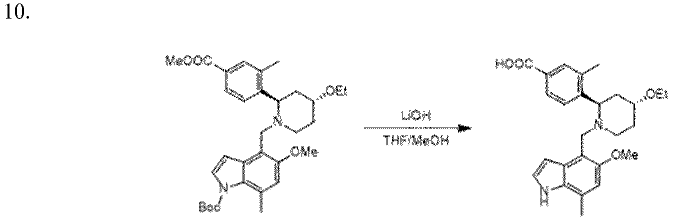

tert-Butyl 4-((2R,4R)-4-ethoxy-2-(4-(methoxycarbonyl)-2-methylphenyl)piperidin-1-yl)methyl)-5-methoxy-7-methyl-1H-indole-1-carboxylate (210 mg, 0.381 mmol) and lithium hydroxide (101 mg, 4.19 mmol) were dissolved in tetrahydrofuran (2.3 mL) and water (2.3 mL), and the reaction was carried out at 50-55 °C for 5.5 h. After completion of the reaction, the pH of the mixture was adjusted to 7-8 with HCl (1M), and the mixture was concentrated under reduced pressure to give a crude product. The crude product was purified by preparative HPLC to afford 4-((2R,4R)-4-ethoxy-1-((5-methoxy-7-methyl-1H-indol-4-yl)methyl)piperidin-2-yl)-3-methylbenzoic acid (Compound II, 68.8 mg, yield 41.2%). Preparation conditions, column: Waters XBridge, 150 × 25 mm, 10 µm; Water (0.1% NH₄HCO₃); Mobile phase B: acetonitrile; Gradient; 16% B to 46% B over 10 min; Flow rate: 40 mL/min; Wavelength: 254 nm / 220 nm; RT = 12 min.

LCMS (ESI, m/z): [M+H] ⁺ = 437.2.

¹H NMR (400 MHz, CDCl3) δ 9.75-9.25 (broad, 1H), 8.25 (s, 1H), 7.95 (s, 1H), 7.75-7.45 (broad, 1H), 7.01 (s, 1H), 6.55 (s, 1H), 6.34 (s, 1H), 4.50-4.45 (m, 2H), 3.72-3.55 (m, 7H), 3.52-3.30 (m, 4H), 3.01 (s, 1H), 2.41 (s, 6H), 2.05-1.75 (m, 2H), 1.22 (s, 3H).

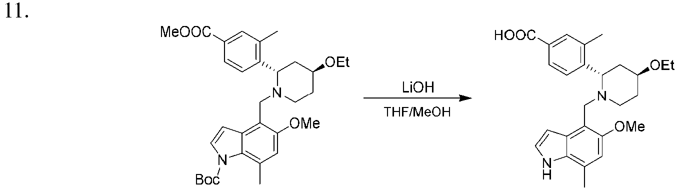

tert-Butyl 4-((2S,4S)-4-ethoxy-2-(4-(methoxycarbonyl)-2-methylphenyl)piperidin-1-yl)methyl)-5-methoxy-7-methyl-1H-indole-1-carboxylate (163 mg, 0.296 mmol) and lithium hydroxide (77.9 mg, 3.26 mmol) were dissolved in tetrahydrofuran (1.4 mL) and water (1.4 mL), and the reaction was carried out at 50-55 °C for 5.5 h. After completion of the reaction, the pH of the mixture was adjusted to 7-8 with HCl (1M), and the mixture was concentrated under reduced pressure to give a crude product. The crude product was purified by preparative HPLC to afford 4-((2S,4S)-4-ethoxy-1-((5-methoxy-7-methyl-1H-indol-4-yl)methyl)piperidin-2-yl)-3-methylbenzoic acid (Compound I, 88.4 mg, yield 68.1%). Preparation conditions, column: Waters XBridge, 150 × 25 mm, 10 µm; Mobile Phase A: Water (0.1% NH₄HCO₃); Mobile phase B: acetonitrile; Gradient; 15% B to 45% B over 10 min; Flow rate: 40 mL/min; Wavelength: 254 nm / 220 nm; RT = 12 min.

LCMS (ESI, m/z): [M+H] ⁺ = 437.2.

¹H NMR (400 MHz, CDCl3) δ 9.75-9.25 (broad, 1H), 8.25 (s, 1H), 7.95 (s, 1H), 7.75-7.45 (broad, 1H), 7.01 (s, 1H), 6.55 (s, 1H), 6.34 (s, 1H), 4.50-4.45 (m, 2H), 3.72-3.55 (m, 7H), 3.52-3.30 (m, 4H), 3.01 (s, 1H), 2.41 (s, 6H), 2.05-1.75 (m, 2H), 1.22 (s, 3H).

### Example 2

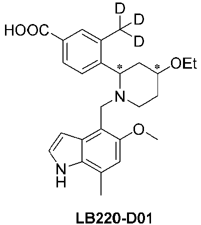

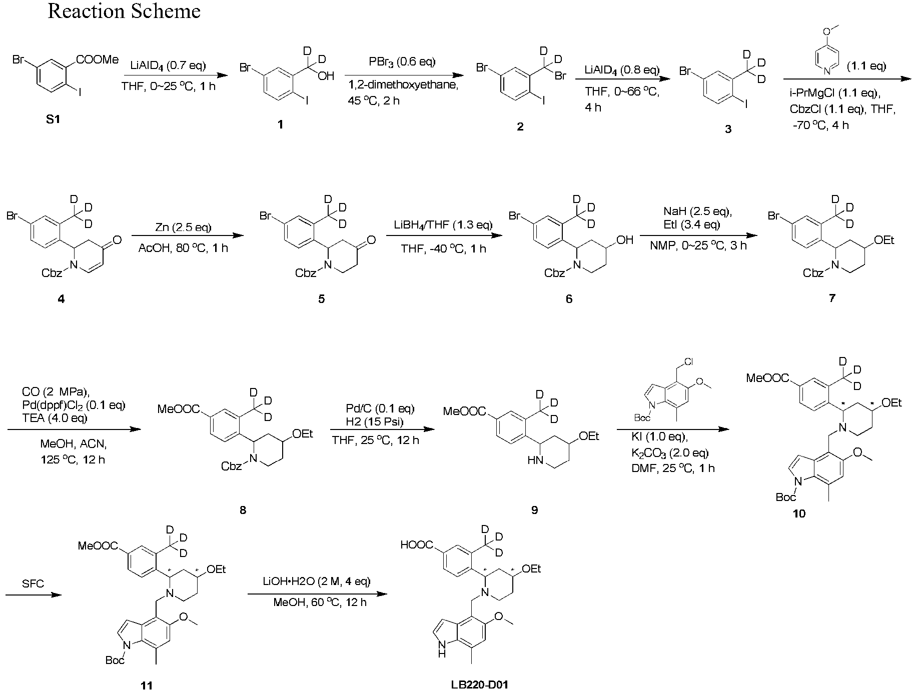

### Experimental Procedure

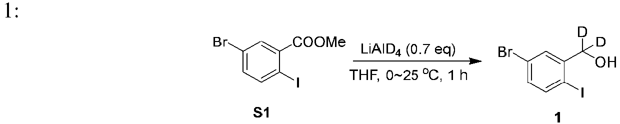

Compound S1 (46.9 g, 138 mmol) was added to anhydrous tetrahydrofuran (500 mL), and the reaction was cooled to 0 °C. LiAlD₄ (3.46 g, 4.71 mL) was added dropwise to the solution, and the reaction was carried out at 25 °C for 1 h. Complete consumption of the starting material was confirmed by TLC (ethyl acetate: petroleum ether = 20:1). Water (50.0 mL) was slowly added dropwise to the reaction solution at 0-10 °C. The mixture was extracted with ethyl acetate (100 mL × 2). The organic phases were concentrated directly, and the residue was purified by column chromatography (SiO₂, petroleum ether/ethyl acetate = 10: 1 to 3: 1), to afford compound 1 (19.0 g, yield 43.8%).

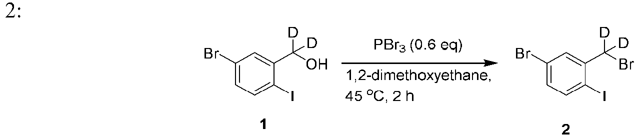

Compound 1 (17.9 g, 56.8 mmol) was added to ethylene glycol dimethyl ether (200 mL), and the resulting solution was cooled to 0 °C. Boron tribromide (9.23 g, 34.1 mmol) was added. The reaction was carried out for 4 h at 45 °C. Complete consumption of the starting material was confirmed by TLC (ethyl acetate: petroleum ether = 20:1). Saturated aqueous sodium bicarbonate solution (100 mL) and water (50.0 mL) were slowly added dropwise to the reaction mixture at 0-10 °C. The mixture was extracted with ethyl acetate (100 mL × 3), and the organic phases were concentrated directly to afford compound 2 (21.0 g, yield 97.7%).

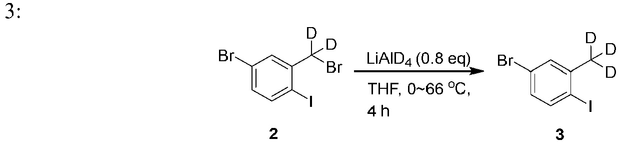

Compound 2 (21.0 g, 55.5 mmol) was added to anhydrous tetrahydrofuran (220 mL), and the reaction was cooled to 0 °C. LiAlD₄ (1.87 g, 44.4 mmol) was added dropwise to the solution, and the reaction was carried out at 25 °C for 0.5 h, and then heated to 66 °C for 3 h. Complete consumption of the starting material was confirmed by TLC (ethyl acetate: petroleum ether = 0: 1). Water (20.0 mL) was slowly added dropwise to the reaction solution while maintaining the temperature at 0 °C. The mixture was extracted with ethyl acetate (100 mL × 2 ). The organic phases were concentrated directly, and the residue was purified by column chromatography (SiO₂, petroleum ether/ethyl acetate = 100: 1 to 10: 1), to afford compound 3 (19.0 g, crude product).

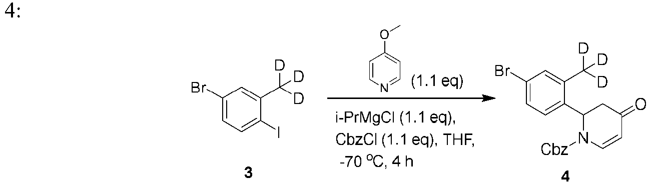

3 (16.5 g, 55.0 mmol) was added to anhydrous tetrahydrofuran (170 mL), and the reaction mixture was cooled to -70 to -60 °C. Isopropylmagnesium chloride-lithium chloride complex (1.30 M, 46.5 mL) was added dropwise, The reaction was carried out at this temperature for 1 h. Then 4-methoxypyridine (6.60 g, 60.5 mmol) and benzyl chloroformate (10.3 g, 60.5 mmol) were added, then the system was reacted at -70 to -60 °C for 1 h. Complete consumption of the starting material was confirmed by TLC (ethyl acetate: petroleum ether = 5:1). Hydrochloric acid (5 M, 50.0 mL) was added to the reaction solution, and the resulting mixture was extracted with ethyl acetate (200 mL × 3). The extracts were washed with saturated aqueous sodium bicarbonate (500 mL × 2). The combined organic phases were dried over anhydrous sodium sulfate, filtered, and concentrated under reduced pressure. The residue was purified by column chromatography (SiO₂, petroleum ether/ethyl acetate = 100: 1 to 3: 1), to afford compound 4 (20.0 g, yield 87.8%).

LCMS (ESI, m/z): [M+H]⁺ = 404.9.

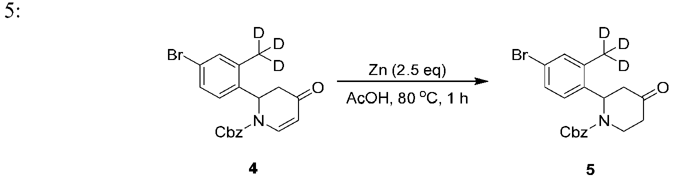

Compound 4 (7.20 g, 17.8 mmol) was added to acetic acid (30.0 mL). The reaction mixture was warmed to 30-40 °C, and zinc powder (3.57 g, 49.1 mmol) was added. The reaction was carried out at 80 °C for 1 h. Complete consumption of the starting material was confirmed by ¹H NMR analysis. The reaction mixture was filtered, and the filter cake was washed with ethyl acetate (100 mL × 1). The filtrate was diluted with ethyl acetate (300 mL). The combined organic phase was washed with saturated aqueous sodium bicarbonate solution (500 mL × 3), dried over anhydrous sodium sulfate, filtered, and concentrated under reduced pressure to afford compound 5 (6.68 g, yield 92.3%).

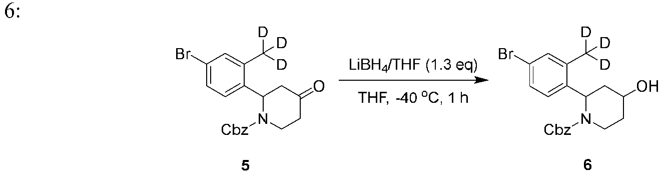

Compound 5 (6.68 g, 16.4 mmol) was added to anhydrous tetrahydrofuran (70.0 mL), and the reatcion mixture was cooled to -40 °C. Lithium borohydride in tetrahydrofuran (2 M, 10.7 mL) was added dropwise, and the reaction was carried out at -40 °C for 1 h Complete consumption of the starting material was confirmed by TLC (ethyl acetate: petroleum ether = 5:1). Aqueous ammonium chloride solution (50.0 mL) was slowly added dropwise to the reaction solution at 0 °C. The reaction mixture was extracted with ethyl acetate (100 mL × 3). The organic phases were concentrated directly, and the residue was purified by column chromatography (SiO₂, petroleum ether/ethyl acetate = 100: 1 to 50: 1), to afford compound 6 (5.70 g, yield 84.8%).

LCMS (ESI, m/z): [M+H]⁺ = 409.

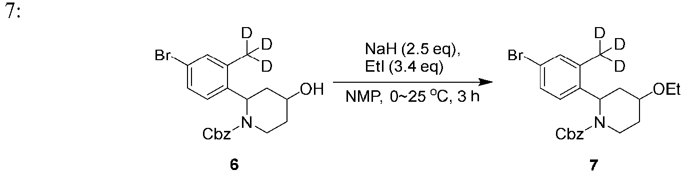

6 (2.00 g, 4.91 mmol) was added to 1-methyl-2-pyrrolidinone (20.0 mL). Sodium hydride (490 mg, 12.2 mmol, 60% purity) was added at 0 °C under a nitrogen atmosphere, and the reaction was carried out at 25 °C for 1 h. Then ethyl iodide (2.68 g, 17.1 mmol) was added at 0-5 °C under nitrogen. The reaction was carried out at 25 °C for 3 h. TLC analysis (ethyl acetate/petroleum ether = 5:1) showed complete consumption of the starting material. Aqueous ammonium chloride solution (10.0 mL) was added to the reaction solution at 0 °C. Then the reaction solution was extracted with ethyl acetate (50 mL × 3), and the organic phase was dried over anhydrous sodium sulfate, filtered, and concentrated under reduced pressure. The residue was purified by column chromatography to afford compound 7 (1.66 g, yield: 77.6% ).

LCMS (ESI, m/z): [M+H]⁺ = 391.1.

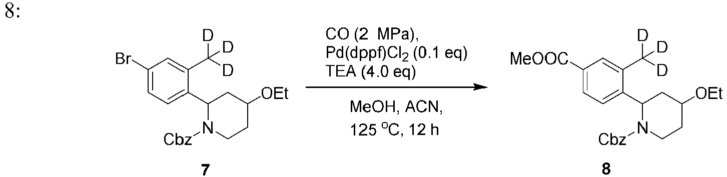

Bis(triphenylphosphine)palladium(II) dichloride (311 mg, 381 µmol) was added to anhydrous methanol (40.0 mL) and acetonitrile (10.0 mL), followed by the sequential addition of compound 7 (1.66 g, 3.81 mmol) and triethylamine (1.54 g, 15.2 mmol). The reaction mixture was purged with Ar three times and then with CO three times. The system was heated at 125 °C and stirred under a CO atmosphere (2 Mpa). The reaction was carried out for 12 h. TLC analysis (petroleum ether/ethyl acetate= 5:1) showed complete consumption of the starting material. The reaction solution was filtered, and the filtrate was concentrated under reduced pressure. The residue was purified by column chromatography (SiO₂, petroleum ether/ethyl acetate = 100:1 to 3:1) to afford compound 8 (1.11 g, yield 70.3%).

LCMS (ESI, m/z): [M+H]⁺ = 415.3.

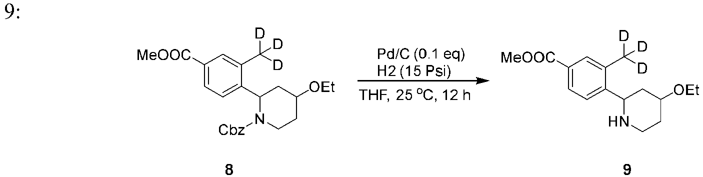

Compound 8 (1.10 g, 2.65 mmol) was dissolved in anhydrous ethanol (10.0 mL) and tetrahydrofuran (10.0 mL), and Pd/C (2.82 g, 10.0%) was added under an argon atmosphere. The reaction mixture was purged with Ar three times and then with H₂ three times. Then the reaction was carried out under H₂ (15 psi )at 25 °C for 12 h. The reaction was monitored by LCMS, and formation of compound **9** was observed. The reaction solution was filtered, and then concentrated under reduced pressure to afford compound 9 (700 mg, yield 97.0%).

LCMS (ESI, m/z): [M+H]⁺ = 28 1.0.

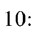

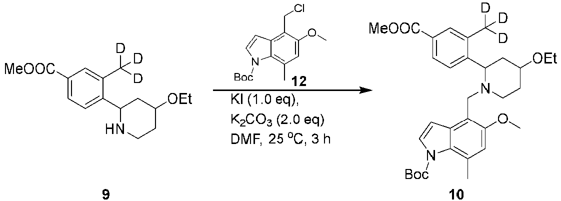

Compound 9 (700 mg, 2.50 mmol) was dissolved in N,N-dimethylformamide (10.0 mL), followed by sequential addition of potassium iodide (414 mg, 2.50 mmol), potassium carbonate (6.90 mg, 4.99 mmol), and compound 12 (1.16 g, 3.74 mmol). The reaction was carried out at room temperature for 2 h. The reaction was monitored by TLC (petroleum ether: ethyl acetate = 3:1)., and formation of compound **10** was observed. Water (20.0 mL) was then added to the reaction system, and the solution was extracted with ethyl acetate (10.0 mL × 3). The organic phase was washed with brine (20.0 mL× 1) and separated. Then the organic phase was dried over anhydrous sodium sulfate, filtered, and concentrated under reduced pressure. The residue was purified by column chromatography (SiO₂, petroleum ether/ethyl acetate = 100: 1 to 4: 1), to afford compound 10 (500 mg, yield: 91.7%).

LCMS (ESI, m/z): [M+H]⁺ = 554.7.

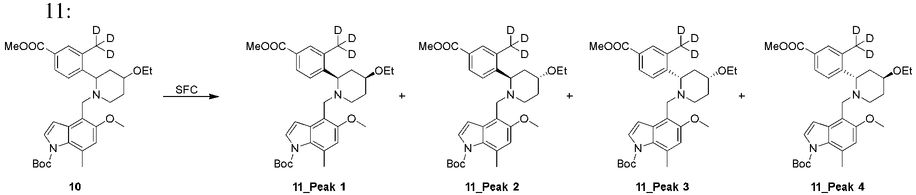

Compound 10 was further separated by chiral SFC using the following method: column: DAICEL CHIRALPAK AD (250 mm × 30 mm, 10 µm); mobile phase: [CO₂-isopropanol]; B%: 25%, isocratic elution; column: DAICEL CHIRALPAK AD (250 mm × 30 mm, 10 µm); mobile phase: [CO₂-EtOH]; B%: 35%, isocratic elution; and column: DAICEL CHIRALPAK AD (250 mm × 30 mm, 10 µm); mobile phase: [CO₂-isopropanol]; B = 30%, isocratic elution, to afford compound 11_Peak 1 (30.0 mg, yield: 6.00%, retention time = 3.5 min), compound 11_Peak 2 (30.0 mg, yield: 6.00%, retention time = 5.5 min), compound 11_Peak 3 (180 mg, yield: 36.0%, retention time = 12.5 min), and compound 11_Peak 4 (200 mg, yield: 40.0%, retention time = 15.5 min).

LCMS (ESI, m/z): [M+H]⁺ = 554.3.

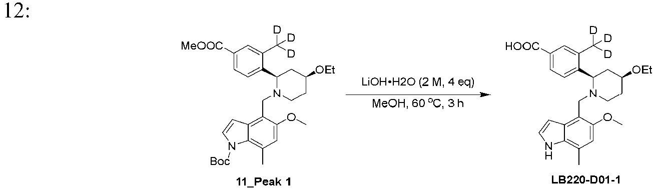

Compound 11_Peak 1 (30.0 mg, 54.1 µmol) was added to anhydrous methanol (0.50 mL) and water (0.10 mL), followed by lithium hydroxide monohydrate (11.3 mg, 270 µmol). The reaction mixture was heated to 60 °C. The reaction was carried out for 12 h. LCMS analysis indicated complete consumption of the starting material. The reaction mixture was concentrated under reduced pressure to remove methanol. The aqueous phase was adjusted to pH 7 with hydrochloric acid (0.5 M) and extracted with ethyl acetate (10.0 mL × 3). The combined organic phases were concentrated directly under reduced pressure. The residue was purified by neutral reversed-phase HPLC (column: CD24-WePure Biotech XPT C18, 150×25 mm, 7 µm; Mobile phase: [H₂O(10mM NH₄HCO₃)-ACN]; Gradient: 12%-42% B over 11.0 min, to afford compound LB220-D01-1 (6.86 mg, yield: 28.6%).

LCMS (ESI, m/z): [M+H]⁺ = 440.3.

¹H NMR: EC19837-666-P1A1 (400 MHz, CHLOROFORM-d) δ = 9.93 - 8.92 (m, 1H), 8.38 - 8.12 (m, 1H), 7.97 - 7.86 (m, 1H), 7.80 - 7.49 (m, 1H), 7.05 - 6.92 (m, 1H), 6.59 - 6.48 (m, 1H), 6.35 (br s, 1H), 4.51 - 4.36 (m, 1H), 4.30 - 4.16 (m, 1H), 3.78 - 3.71 (m, 1H), 3.66 (br s, 3H), 3.50 - 3.44 (m, 2H), 3.36 (br s, 1H), 2.98 (br d, J = 2.0 Hz, 1H), 2.39 (br s, 5H), 2.00 (br d, J = 12.5 Hz, 1H), 1.84 - 1.75 (m, 1H), 1.31 - 1.17 (m, 4H).

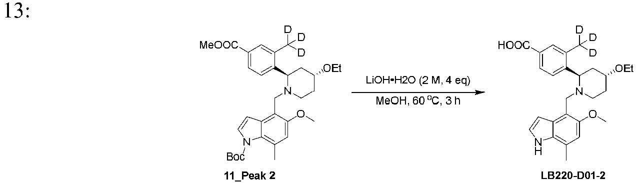

Compound 11_Peak 2 (30.0 mg, 54.1 µmol) was added to anhydrous methanol (0.50 mL) and water (0.10 mL), followed by lithium hydroxide monohydrate (11.3 mg, 270 µmol). The reaction mixture was heated to 60 °C. The reaction was carried out for 12 h. LCMS analysis indicated complete consumption of the starting material. The reaction mixture was concentrated under reduced pressure to remove methanol. The aqueous phase was adjusted to pH 7 with hydrochloric acid (0.5 M) and extracted with ethyl acetate (10.0 mL × 3). The combined organic lphases were concentrated directly under reduced pressure. The residue was purified by neutral reversed-phase HPLC (column: CD24-WePure Biotech XPT C18, 150×25 mm, 7 µm; Mobile phase: [H₂O(10mM NH₄HCO₃)-ACN]; Gradient: 12%-42% B over 11.0 min, to afford compound LB220-D01-2 (9.81 mg, yield: 40.0%).

LCMS (ESI, m/z): [M+H]⁺ = 440.3.

¹H NMR: EC19837-668-P1A2 (400 MHz, DMSO-d6) δ = 7.93 - 7.79 (m, 2H), 7.78 - 7.70 (m, 1H), 7.27 - 7.19 (m, 1H), 6.68 - 6.61 (m, 1H), 6.45 - 6.35 (m, 1H), 3.89 (br d, J = 10.8 Hz, 1H), 3.70 - 3.65 (m, 3H), 3.64 - 3.53 (m, 2H), 3.47 - 3.36 (m, 2H), 3.31 (br d, J = 11.4 Hz, 1H), 2.55 (br d, J = 12.0 Hz, 2H), 2.39 (s, 3H), 1.82 (br d, J = 12.1 Hz, 1H), 1.69 - 1.54 (m, 2H), 1.25 - 1.18 (m, 1H), 1.16 - 0.99 (m, 3H).

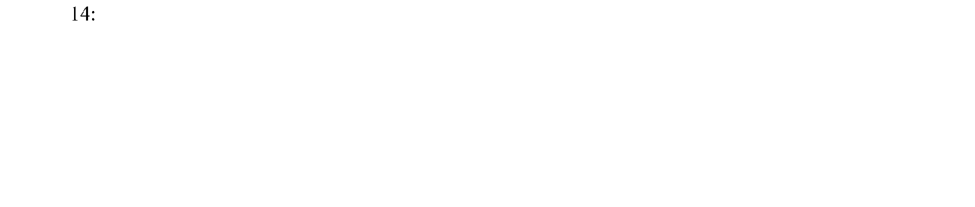

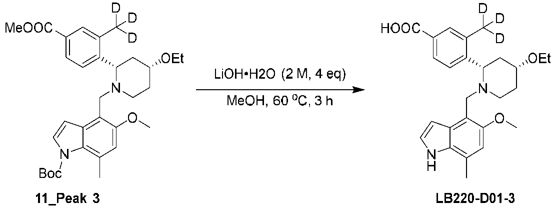

Compound 11_Peak 3 (180 mg, 325 µmol) was added to anhydrous methanol (1.00 mL) and water (0.50 mL), followed by lithium hydroxide monohydrate (68.2 mg, 1.63 mmol). The reaction mixture was heated to 60 °C. The reaction was carried out for 12 h. LCMS analysis indicated complete consumption of the starting material. The reaction mixture was concentrated under reduced pressure to remove methanol. The aqueous phase was adjusted to pH 7 with hydrochloric acid (0.5 M) and extracted with ethyl acetate (10.0 mL × 3). The combined organic phases were concentrated directly under reduced pressure. The residue was purified by neutral reversed-phase HPLC (column: CD24-WePure Biotech XPT C18, 150×25 mm, 7 µm; Mobile phase: [H₂O(10mM NH₄HCO₃)-ACN]; Gradient: 12%-42% B over 11.0 min, to afford compound LB220-D01-3 (52.4 mg, yield: 36.0%).

LCMS (ESI, m/z): [M+H]⁺ = 440.3.

¹H NMR: EC19837-665-P1A (400 MHz, DMSO-d6) δ = 10.85 - 10.77 (m, 1H), 7.90 - 7.80 (m, 2H), 7.80 - 7.73 (m, 1H), 7.28 - 7.22 (m, 1H), 6.68 - 6.61 (m, 1H), 6.43 - 6.36 (m, 1H), 3.69 (s, 3H), 3.55 - 3.51 (m, 1H), 3.47 (br s, 1H), 3.45 - 3.39 (m, 3H), 3.15 (br d, J = 11.8 Hz, 1H), 2.79 (br d, J = 12.1 Hz, 1H), 2.44 - 2.38 (m, 3H), 2.10 - 2.01 (m, 1H), 1.97 (br d, J = 11.0 Hz, 1H), 1.88 - 1.80 (m, 1H), 1.40 - 1.30 (m, 1H), 1.25 - 1.15 (m, 1H), 1.07 - 1.01 (m, 3H).

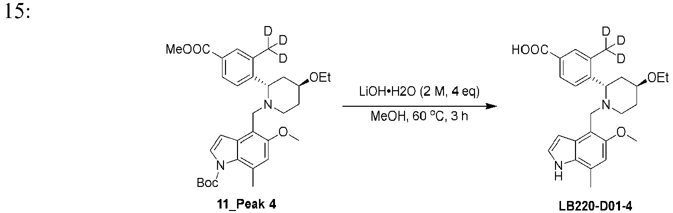

Compound 11_Peak 4 (200 mg, 331 µmol) was added to anhydrous methanol (1.00 mL) and water (0.50 mL), followed by lithium hydroxide monohydrate (75.7 mg, 1.81 mmol). The reaction mixture was heated to 60 °C. The reaction was carried out for 12 h. LCMS analysis indicated complete consumption of the starting material. The reaction mixture was concentrated under reduced pressure to remove methanol. The aqueous phase was adjusted to pH 7 with hydrochloric acid (0.5 M) and extracted with ethyl acetate (10.0 mL × 3). The combined organic phases were concentrated directly under reduced pressure. The residue was purified by neutral reversed-phase HPLC (column: CD24-WePure Biotech XPT C18, 150×25 mm, 7 µm; Mobile phase: [H₂O(10mM NH₄HCO₃)-ACN]; Gradient: 12%-42% B over 11.0 min, to afford compound LB220-D01-4 (59.8 mg, yield: 37.5%).

LCMS (ESI, m/z): [M+H]⁺ = 440.3.

¹H NMR: EC19837-665-P1A (400 MHz, CHLOROFORM-d) δ = 8.05 - 7.97 (m, 2H), 7.94 (br s, 1H), 7.91 (s, 1H), 7.21 - 7.17 (m, 1H), 6.70 - 6.68 (m, 1H), 6.65 (br s, 1H), 3.78 (s, 4H), 3.51 (q, J = 7.0 Hz, 3H), 3.44 - 3.37 (m, 1H), 3.29 (br d, J = 12.4 Hz, 1H), 3.05 (br d, J = 11.8 Hz, 1H), 2.46 (s, 3H), 2.15 (br d, J = 12.6 Hz, 1H), 2.09 - 2.04 (m, 1H), 1.92 (br d, J = 12.1 Hz, 1H), 1.64 - 1.50 (m, 2H), 1.17 (t, J = 7.0 Hz, 3H).

### Example 3

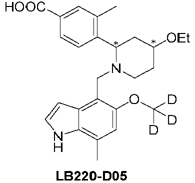

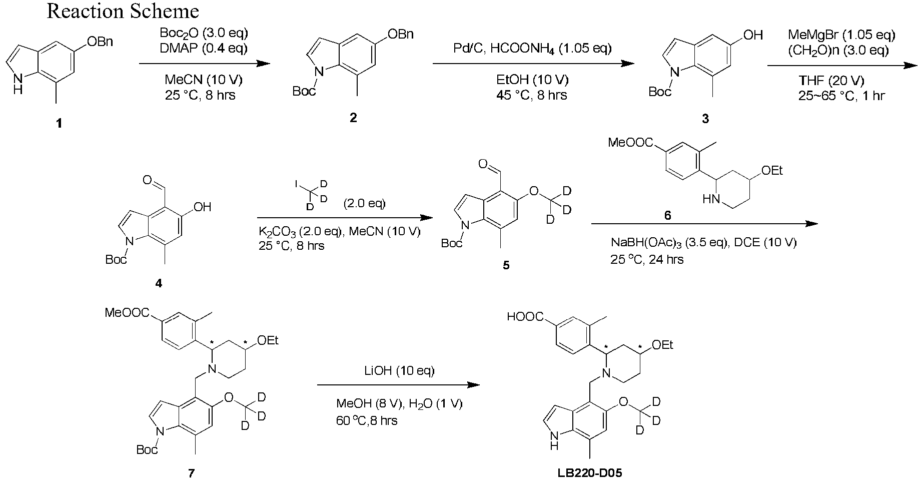

### Experimental Procedure

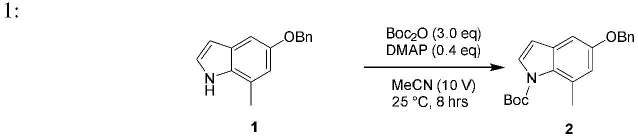

Compound 1 (2.00 g, 8.43 mmol, 1.00 eq) was dissolved in MeCN (20.0 mL). Boc₂O (5.52 g, 25.3 mmol, 5.81 mL, 3.00 eq) and DMAP (412 mg, 3.37 mmol, 0.40 eq) were added at 25 °C. The reaction was carried out at 25 °C for 8 h. Complete consumption of the starting material was confirmed on a TLC plate (SiO₂, petroleum ether/ethyl acetate= 10:1). Saturated aqueous NH₄Cl solution (20.0 mL) was added to the reaction solution at 25 °C, and the mixture was extracted with ethyl acetate (20 mL × 2). The combined organic phase was dried over anhydrous sodium sulfate, filtered, and concentrated under reduced pressure. The residue was purified by column chromatography (SiO₂, petroleum ether/ethyl acetate = 20: 1 to 10: 1), to afford compound 2 (2.80 g, yield: 98.5%).

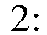

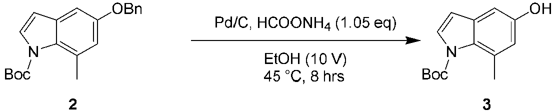

Compound 2 (2.80 g, 8.30 mmol, 1.00 eq) was dissolved in EtOH (25.0 mL). Pd/C (883 mg, 830 µmol, 10.0% purity, 0.10 eq) and HCOONH₄ (549 mg, 8.71 mmol, 1.05 eq) were added at 25 °C. The reaction was carried out at 45 °C for 3 h. Complete consumption of the starting material was confirmed on a TLC plate (SiO₂, petroleum ether/ethyl acetate= 10:1). The reaction solution was filtered through Celite, and the filter cake was washed with MeOH (50.0 mL). The combined organic phase was concentrated under reduced pressure to afford crude compound 3 (2.10 g, crude product).

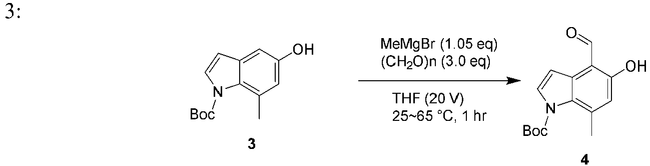

Compound 3 (1.60 g, 6.47 mmol, 1.00 eq) was dissolved in THF (30.0 mL). MeMgBr (3.00 M, 2.26 mL, 1.05 eq) was added dropwise at 20-25 °C. The reaction was carried out at 45 °C for 0.5 h. Then paraformaldehyde (640 mg, 19.4 mmol, 3.00 eq) was added, and the reaction was carried out at 65 °C for 1 h. Complete consumption of the starting material was confirmed on a TLC plate (SiO₂, petroleum ether/ethyl acetate= 10: 1, R_{f} = 0.66). Saturated aqueous NH₄Cl solution (20.0 mL) was added slowly to the reaction solution at 25 °C, and the mixture was extracted with ethyl acetate (20 mL × 2). The combined organic phase was dried over anhydrous sodium sulfate, filtered, and concentrated under reduced pressure. The residue was purified by column chromatography (SiO₂, petroleum ether/ethyl acetate = 1: 0 to 100: 1), to afford compound 4 as a yellow solid (640 mg, yield: 35.9%).

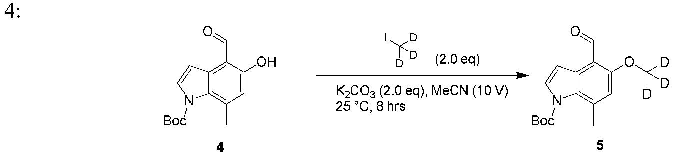

Compound 4 (640 mg, 2.32 mmol, 1.00 eq) and K₂CO₃ (643 mg, 4.65 mmol, 2.00 eq) were dissolved in MeCN (7.00 mL). CD₃I (674 mg, 4.65 mmol, 289 µL, 2.00 eq) was added at 25 °C. The reaction was carried out at 25 °C for 8 h. Complete consumption of the starting material was confirmed on a TLC plate (SiO₂, petroleum ether/ethyl acetate= 20:1). Water (10.0 mL) was added to the reaction solution at 25 °C, and the mixture was extracted with ethyl acetate (10 mL × 2). The combined organic phase was dried over anhydrous sodium sulfate, filtered, and concentrated under reduced pressure. The residue was purified by column chromatography (SiO₂, petroleum ether/ethyl acetate = 20: 1), to afford compound 5 (600 mg, yield: 88.3%).

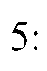

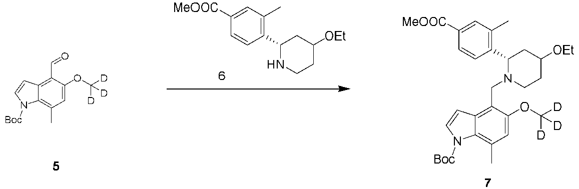

Compounds 6 (600 mg, 2.05 mmol, 1.00 equiv) and 5 (569 mg, 2.05 mmol, 1.00 eq) were dissolved in DCE (10.0 mL). MgSO₄ (741 mg, 6.16 mmol, 3.00 eq) was added at 25 °C. Then the reaction was carried out at 25 °C for 12 h. NaBH(OAc)₃ (1.52 g, 7.18 mmol, 3.50 eq) was then added at 25 °C, and the reaction was carried out at 25 °C for 12 h. LCMS analysis indicated complete consumption of the starting material. The reaction solution was filtered through Celite, and the filter cake was washed with EtOAc (20.0 mL). The combined organic phase was washed with saturated aqueous NaHCO₃ solution (15.0 mL). The organic phase was washed with water (10.0 mL), dried over anhydrous sodium sulfate, filtered, and concentrated under reduced pressure. The residue was purified by neutral reversed-phase HPLC (column: Phenomenex luna C18, 100×40 mm, 3 µm; Mobile phase: [H₂O(0.05%HCl)-ACN]; Gradient: 38%-68% B over 15.0 min), to afford compound **7_Peak1** (170 mg; yield: 15.0%; retention time: 4.8 min). to afford compound **7_Peak2** (209 mg; yield: 16.7%; retention time: 8.5 min). to afford compound **LB220-D05-7_Peak3** (32.0 mg; yield: 2.82%; retention time: 13.4 min).

LCMS (ESI, m/z): [M+H]⁺ = 554.4.

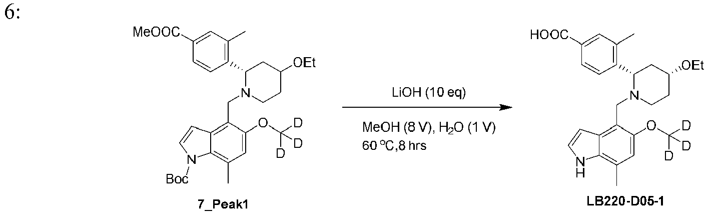

Compound **7_Peak1** (170 mg, 307 µmol, 1.00 eq) was dissolved in MeOH (5.00 mL) and H₂O (1.00 mL). Lithium hydroxide monohydrate (129 mg, 3.07 mmol, 10.0 eq) was added, and the reaction was carried out at 60 °C for 8 h. LCMS analysis indicated complete consumption of the starting material. The reaction solution was adjusted to pH 7-8 with hydrochloric acid (2.00 M) and concentrated under reduced pressure. The residue was purified by neutral reversed-phase HPLC (column: CD07-Daisogel SP-100-8-ODS-PK, 150×25 mm, 10 µm; Mobile phase: [H₂O(10mM NH₄HCO₃)-ACN]; Gradient: 15%-45% B over 10.0 min, to afford compound **LB220-D05-1** (40.53 mg, yield: 29.1%).

¹H NMR: (400 MHz, MeOD) *δ* 8.02 (d, *J =* 8.0 Hz, 1H), 7.93 (s, 1H), 7.67 (d, *J =* 8.0 Hz, 1H), 7.30 (d, *J* = 3.2 Hz, 1H), 6.75 (s, 1H), 6.32 (d, *J =* 2.8 Hz, 1H), 4.59-4.64 (m, 2H), 4.24-4.27 (m, 1H), 4.00-4.03 (m, 1H), 3.72-3.78 (m, 1H), 3.55-3.60 (m, 2H), 3.47-3.50 (m, 1H), 2.55 (s, 3H), 2.50 (s, 3H), 2.30-2.34 (m, 1H), 2.17-2.20 (m, 1H), 1.83-1.93 (m, 1H), 1.69-1.77 (m, 1H), 1.16 (t, *J =* 7.0 Hz, 3H).

LCMS (ESI, m/z): [M+H]⁺ = 440.2.

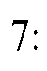

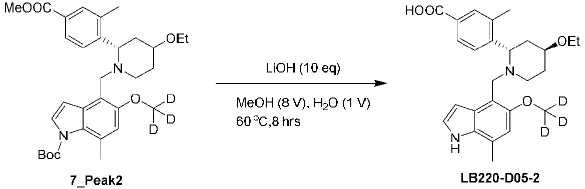

Compound **7_Peak2** (209 mg, 3.77 µmol, 1.00 eq) was dissolved in MeOH (5.00 mL) and H₂O (1.00 mL). Lithium hydroxide monohydrate (158 mg, 3.77 mmol, 10.0 eq) was added, and the reaction was carried out at 60 °C for 8 h. LCMS analysis indicated complete consumption of the starting material. The reaction solution was adjusted to pH 7-8 with hydrochloric acid (2.00 M) and concentrated under reduced pressure. The residue was purified by neutral reversed-phase HPLC (column: CD07-Daisogel SP-100-8-ODS-PK, 150×25 mm, 10 µm; Mobile phase: [H₂O(10mM NH₄HCO₃)-ACN]; Gradient: 15%-45% B over 10.0 min, to afford compound **LB220-D05-2** (64.40 mg, yield: 38.8%).

¹H NMR: (400 MHz, MeOD) *δ* 8.02 (d, *J =* 8.0 Hz, 1H), 7.91 (s, 1H), 7.65 (d, *J =* 8.0 Hz, 1H), 7.31 (d, *J* = 3.2 Hz, 1H), 6.75 (s, 1H), 6.40 (d, *J =* 2.4 Hz, 1H), 4.59 (s, 1H), 4.29-4.32 (m, 1H), 4.16-4.19 (m, 1H), 3.80 (s, 1H), 3.59-3.64 (m, 2H), 3.53-3.56 (m, 1H), 3.36 (s, 1H), 2.53 (s, 3H), 2.50 (s, 3H), 2.10-2.21 (m, 2H), 1.98-2.06 (m, 2H), 1.31 (t, *J =* 7.0 Hz, 3H).

LCMS (ESI, m/z): [M+H]⁺ = 440.2.

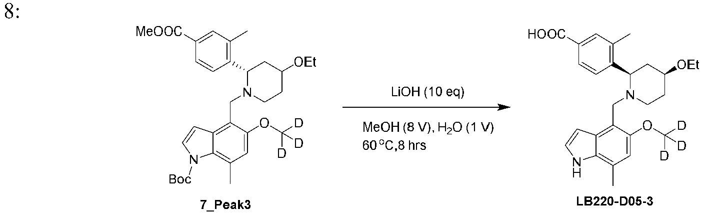

Compound **7_Peak3** (32 mg, 57.8 µmol, 1.00 eq) was dissolved in MeOH (5.00 mL) and H₂O (1.00 mL). Lithium hydroxide monohydrate (24.3 mg, 578 µmol, 10.0 eq) was added, and the reaction was carried out at 60 °C for 8 h. LCMS analysis indicated complete consumption of the starting material. The reaction solution was adjusted to pH 7-8 with hydrochloric acid (2.00 M) and concentrated under reduced pressure. The residue was purified by neutral reversed-phase HPLC (column: CD02-Waters Xbidge BEH C18, 150×25 mm, 10 µm; Mobile phase: [H₂O(10mM NH₄HCO₃)-ACN]; Gradient: 15%-45% B over 10.0 min, to afford compound **LB220-D05-3** (64.40 mg, yield: 38.8%).

¹H NMR: (400 MHz, MeOD) *δ* 8.01 (d, *J =* 8.0 Hz, 1H), 7.90 (s, 1H), 7.65 (d, *J =* 8.0 Hz, 1H), 7.30 (d, *J = 3.2* Hz, 1H), 6.74 (s, 1H), 6.40 (d, *J =* 3.2 Hz, 1H), 4.60 (s, 1H), 4.25-4.29 (m, 1H), 4.12-4.15 (m, 1H), 3.79 (s, 1H), 3.56-3.63 (m, 2H), 3.49-3.56 (m, 1H), 3.34 (s, 1H), 2.52 (s, 3H), 2.49 (s, 3H), 2.08-2.18 (m, 2H), 1.98-2.05 (m, 2H), 1.30 (t, *J =* 7.0 Hz, 3H).

LCMS (ESI, m/z): [M+H]⁺ = 440.2.

### Example 4

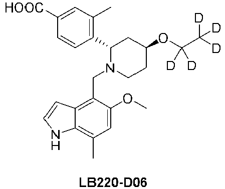

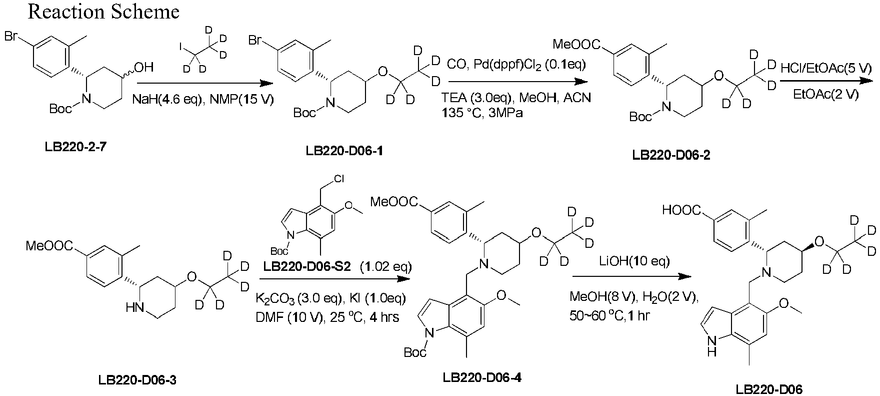

### Experimental Procedure

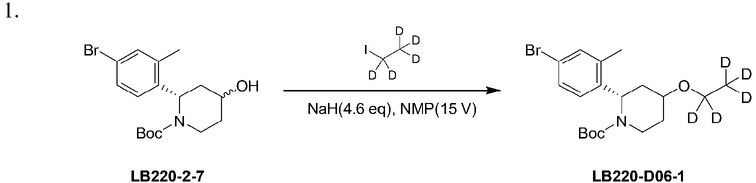

Sodium hydride (600.00 mg, 15.00 mmol, 60% purity, 4.63 eq) was dissolved in N-methylpyrrolidone (12 mL). Compound LB220-2-7 (1.2 g, 3.24 mmol, 1 eq) was dissolved in N-methylpyrrolidone (3 mL), and the resulting solution was added to the reaction solution at 0-5 °C. The reaction was carried out at 0-5 °C for 0.5 h. Deuterated iodoethane (1 g, 6.21 mmol, 1.92 eq) was then added at 0-5 °C. The reaction solution was stirred at 15-25 °C for 4 h. LCMS analysis indicated formation of the desired product LB220-D06-1. Saturated aqueous ammonium chloride solution was added to the reaction mixture at 0-15 °C to adjust the pH to 6-7, and the mixture was stirred for 0.5 h. Then the reaction solution was extracted with ethyl acetate (30 mL × 2). The combined organic phase was concentrated to afford the crude product, The crude product was purified by column chromatography (silica, petroleum ether: ethyl acetate = 1: 0 to 20: 1), to afford compound LB220-D06-1 (0.95 g, yield 72%).

LCMS (ESI, m/z): [M+H]⁺ = 403.1.

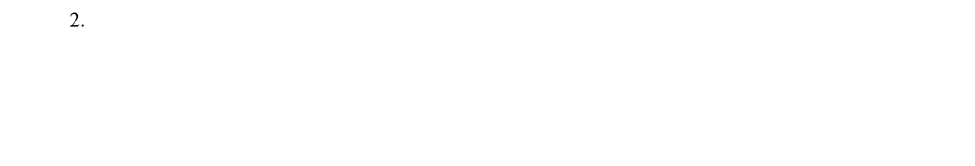

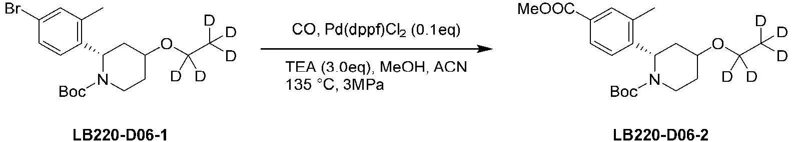

Triethylamine (790 mg, 7.82 mmol, 1.09 mL, 3.32 eq) was dissolved in methanol (10 mL) and acetonitrile (20 mL). Pd(dppf)Cl₂ (633 mg, 775 µmol, 3.29e-1 eq) and compound LB220-D06-1 (950 mg, 2.36 mmol, 1 eq) were added at 20-30 °C. The reaction was carried out under a carbon monoxide atmosphere (3 MPa) at 125-135 °C for 12 h. LCMS analysis indicated complete consumption of the starting material. The reaction solution was cooled to 20-25 °C and concentrated to remove methanol and acetonitrile. Ethyl acetate (10 mL) and water (10 mL) were added, and the resulting mixture was filtered. The phases were separated, and the aqueous phase was extracted with ethyl acetate (10 mL × 2). The combined organic phase concentrated, and the residue was purified by column chromatography (silica, petroleum ether: ethyl acetate = 50: 0 to 30: 1), to afford compound LB220-D06-2 (710 mg, yield 78.8%).

LCMS (ESI, m/z): [M+H]⁺ = 383.2.

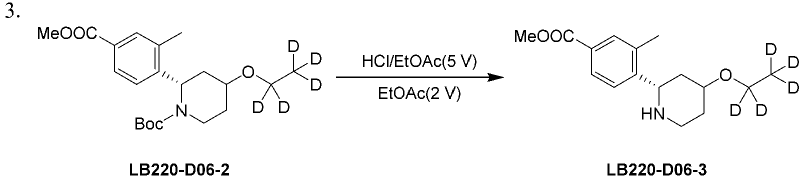

Compound LB220-D06-2 (710 mg, 1.86 mmol, 1.00 eq) was dissolved in ethyl acetate (1.4 mL). Hydrogen chloride in ethyl acetate (2 M, 3.5 mL) was added, and the reaction mixture was stirred at 30-40 °C for 4 h. LCMS analysis indicated complete consumption of the starting material. The reaction solution was concentrated to remove ethyl acetate, affording compound LB220-D06-3 (550 mg, hydrochloride, crude product).

LCMS (ESI, m/z): [M+H]⁺ = 283.2.

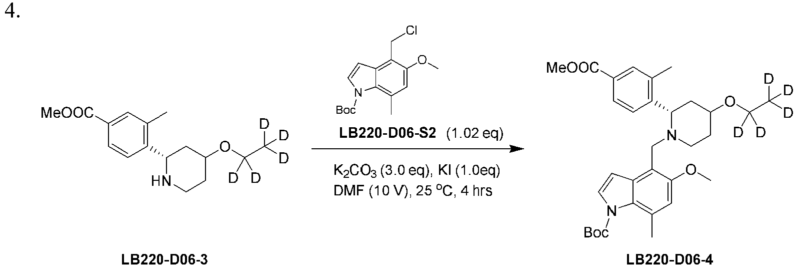

Compound LB220-D06-3 (200 mg, 627 µmol, 1.00 eq , HCl) was dissolved in N,N-dimethylformamide (2.00 mL). Potassium iodide (100 mg, 602 µmol, 1.06 eq), potassium carbonate (260 mg, 1.88 µmol, 3.00 eq), and compound LB220-D06-S2 (200 mg, 645 µmol, 1.03 eq) were added sequentially. The reaction was carried out at 10-20 °C for 4 h. LCMS analysis indicated complete consumption of the starting material. Water (10.0 mL) was added to the reaction system, and the mixture was extracted with ethyl acetate (10.0 mL × 2). The organic phase was dried over anhydrous sodium sulfate, filtered, and concentrated under reduced pressure. The residue was purified on a TLC plate (SiO₂, petroleum ether/ethyl acetate = 3:1), to afford compound **LB220-D06-4** (234 mg, yield 67.1%).

LCMS (ESI, m/z): [M+H]⁺ = 556.3.

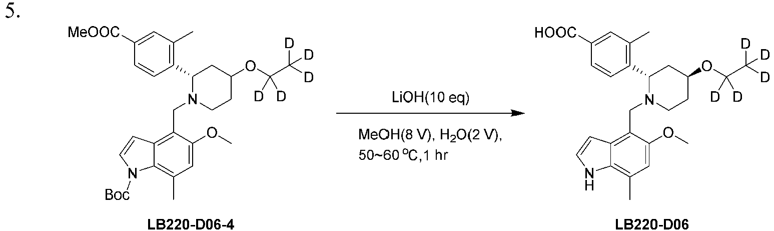

Compound **LB220-D06-4** (234 mg, 421 µmol, 1.00 eq) was dissolved in methanol (2 mL) and H₂O (0.5 mL). Lithium hydroxide monohydrate (177 mg, 4.21 mmol, 10 eq) was added at 15 to 25 °C. The reaction was carried out at 55-65 °C for 1 h. LCMS analysis indicated complete consumption of the starting material. The reaction solution was adjusted to pH 6-7 with hydrochloric acid (0.1M) and concentrated under reduced pressure to afford the crude product. The crude product was purified by reverse-phase HPLC (CD20-Waters Xbidge BEH C18 250×25 mm, 10 µm; mobile phase: [H₂O(10mM NH₄HCO₃)-ACN]; gradient: 15%-45% B over 10.0 min) to afford compound LB220-D06 (48 mg, 99.9% purity).

LCMS (ESI, m/z): [M+H]+= 442.2.

1H NMR: EC20710-463-P1F (400 MHz, DMSO-d6), δ ppm: 10.81 (s, 1 H), 7.95 - 7.89 (m, 1 H), 7.88 - 7.82 (m, 1 H), 7.76 (s, 1 H), 7.25 (t, J=2.80 Hz, 1 H), 6.65 (s, 1 H), 6.49 - 6.40 (m, 1 H), 3.78 - 3.72 (m, 1 H), 3.70 (s, 3 H), 3.59 - 3.53 (m, 2 H), 3.17 (d, J=11.6 Hz, 1 H), 2.54 - 2.51 (m, 1 H), 2.43 (s, 3 H), 2.41 (s, 3 H), 2.39 - 2.30 (m, 1 H), 1.84 - 1.80(m, 1 H), 1.72 - 1.62(m, 1 H), 1.61 - 1.43 (m, 2 H).

### Example 5

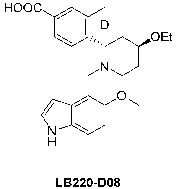

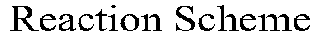

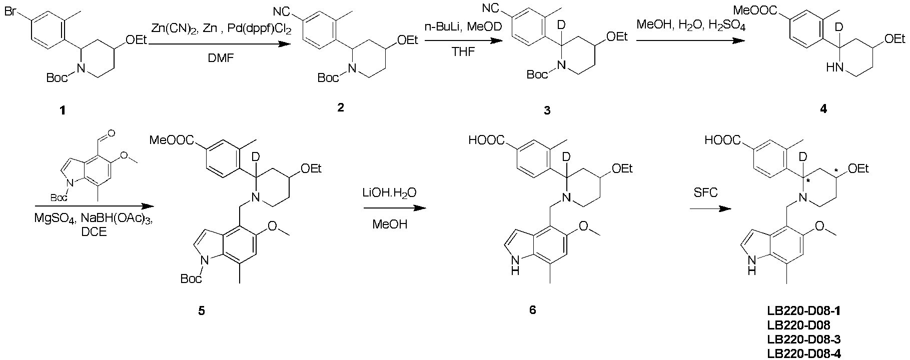

### Experimental Procedure

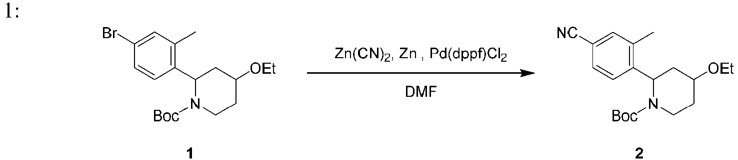

Compound 1 (2.3 g, 5.77 mmol), zinc powder (1.38 g, 21.1 mmol), and zinc cyanide (3.68 g, 31.3 mmol) were added to N,N-dimethylformamide (23 mL). [1,1'-Bis(diphenylphosphino)ferrocene]dichloropalladium(II) (422 mg, 577 µmol) was then added under a nitrogen atmosphere. The reaction solution was heated to 130-135 °C. The reaction was carried out at 130-135 °C for 4 h. TLC analysis (petroleum ether: ethyl acetate= 3:1) showed complete consumption of the starting material. The reaction solution was diluted with water (50 mL) and the aqueous phase was extracted with ethyl acetate (50 mL × 2). The organic phase was washed with saturated brine (50 mL). Then the organic phase was dried over anhydrous sodium sulfate, filtered, and concentrated under reduced pressure. The residue was purified by column chromatography (petroleum ether: ethyl acetate = 100: 20 to 100: 30), to afford compound 2 (610 mg, crude product).

LCMS (ESI, m/z): [M-99]⁺ = 245.1.

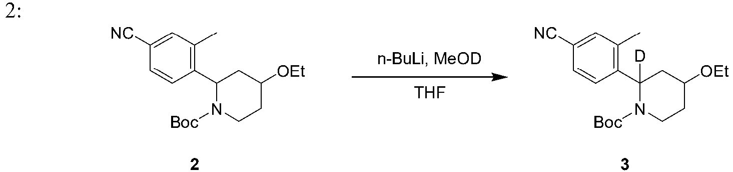

Compound 2 (200 mg, 580 µmol) was dissolved in anhydrous tetrefuran (4 mL), and the solution was cooled to -70 to -60 °C. n-BuLi (2.5 M, 301 µL) was added dropwise under a nitrogen atmosphere. The reaction was carried out at -70 to -60 °C for 1 h, followed by the dropwise addition of deuterated methanol (41.9 mg, 1.16 mmol) at -70 to -60 °C. The reaction was carried out at -70 to -60 °C for 1 h. LCMS analysis indicated complete consumption of the starting compound material. The reaction solution was diluted with water (5 mL) and the aqueous phase was extracted with ethyl acetate (5 mL × 2). The organic phase was washed with saturated brine (5 mL). Then the organic phase was dried over anhydrous sodium sulfate, filtered, and concentrated under reduced pressure. The residue was purified by column chromatography (petroleum ether: ethyl acetate = 100: 20 to 100: 30), to afford compound 3 (670 mg, yield: 47.8%).

LCMS (ESI, m/z): [M-99]⁺ = 246.1.

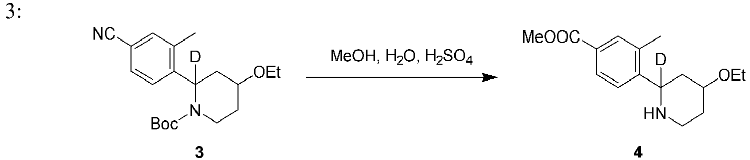

Compound 3 (730 mg, 2.11 mmol) was dissolved in water (7.3 mL) and sulfuric acid (7.3 mL). Then methanol (7.3 mL) was added. The reaction was carried out at 75-80 °C for 24 h. LCMS analysis indicated complete consumption of the starting material. The mixture was adjusted to pH 8-9 with saturated aqueous sodium bicarbonate solution and extracted with ethyl acetate (50 mL × 2). The organic phase was dried over anhydrous sodium sulfate, filtered, and concentrated under reduced pressure to afford compound 4 (520 mg, yield: 88.4%).

LCMS (ESI, m/z): [M+H]⁺ = 279.1.

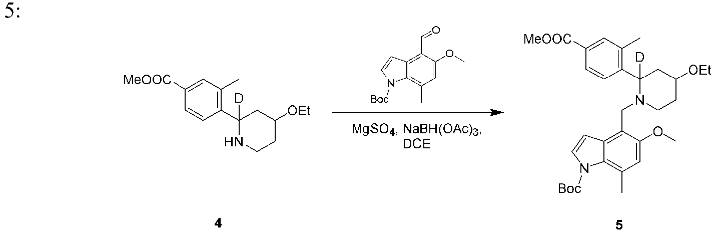

Compound 4 (520 mg, 1.87 mmol) and 5-methoxy-7-methyl-T-BOC-1H-indole-4-carbaldehyde (1.08 g, 3.74 mmol) were dissolved in 1,2-dichloroethane (10 mL). Compound anhydrous magnesium sulfate (674 mg, 5.60 mmol) was added, and the reaction was carried out at 15-25 °C for 12 h. Sodium triacetoxyborohydride (1.39 g, 6.54 mmol) was then added, and the mixture was stirred at 15-25 °C for 12 h. The mixture was filtered, and the filtrate was diluted with saturated aqueous sodium bicarbonate solution (50 mL). The aqueous phase was extracted with ethyl acetate (50 mL × 2). The organic phase was dried over anhydrous sodium sulfate, filtered, and concentrated under reduced pressure. The residue was purified by column chromatography (petroleum ether: ethyl acetate = 100: 20 to 100: 30), to afford compound 5 (690 mg, yield: 66.9%).

LCMS (ESI, m/z): [M+H]⁺ = 552.4.

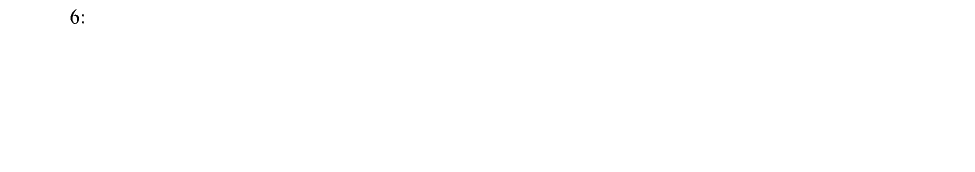

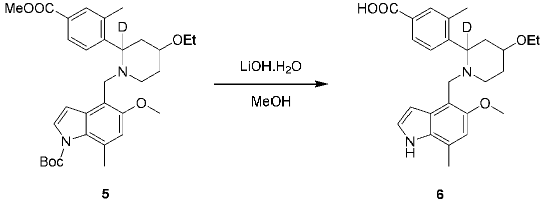

Compound 5 (670 mg, 1.21 mmol) was dissolved in methanol (10 mL), and lithium hydroxide monohydrate (2M, 3.04 mL) was added. The reaction was carried out 60-65 °C for 1 h. LCMS analysis indicated complete consumption of the starting material. The reaction solution was adjusted to pH 6-7 with hydrochloric acid (2 M) and concentrated under reduced pressure. The residue was purified by reverse-phase HPLC (column: CD18-Welch Utimate C18 150 × 40 × 7 um; mobile phase: [H₂O(0.05% HCl)-ACN]; gradient: 7%-37% B over 10.0 min) and reverse-phase HPLC (column: CD09-Phenomenex Gemini C18 150 × 30 × 5 µm; mobile phase: [H₂O(10mM NH₄HCO₃)-ACN]]; gradient: 16%-46% B over 13.0 min) to afford compound 6 (55 mg, yield 10.3%).

LCMS (ESI, m/z): [M+H]⁺ = 438.3.

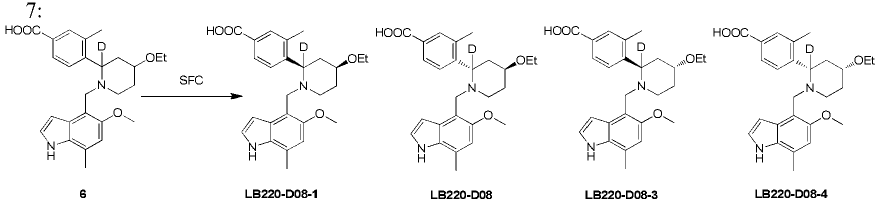

Compound 6 (250 mg) was purified by SFC (column: SFC-IK-30-DAICEL CHIRAL IK (250mm × 30mm, 10 µm); mobile phase: [CO₂-MeOH(0.1%NH₃H₂O), to afford LB220-D08-1 (15 mg), LB220-D08-3 (10 mg), LB220-D08 (150 mg), and LB220-D08-4 (15 mg).

LCMS (ESI, m/z): [M+H]⁺ = 438.3.

### Example 6-10

The following compounds were further synthesized with reference to the preceding examples (except that different starting materials were used, and the preparation procedures may be referred to those described in the preceding examples). The LCMS data for each compound are shown in the table below.

| Example | Structural formula | MS value [M+H⁺] | Example | Structural formula | MS value [M+H⁺] |
|---|---|---|---|---|---|
| 6 | 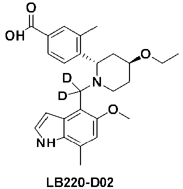 | 439.25 | 7 | 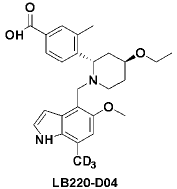 | 440.26 |
| 8 | 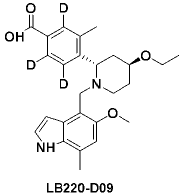 | 440.26 | 9 | 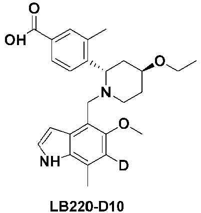 | 438.24 |
| 10 | 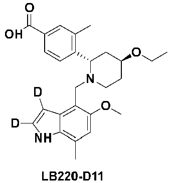 | 439.25 | | | |

### Comparative Example

This comparative example provides compounds D1 and D2, the structures of which are shown below:

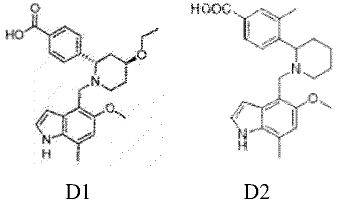

D1 was purchased from MCE, and D2 was prepared in-house according to the following procedure:

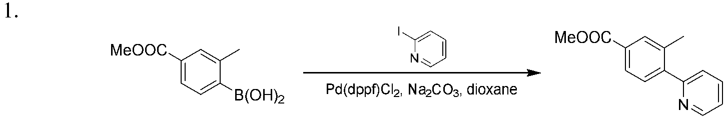

Pd(dppf)Cl₂ (100 mg, 137 µmol, 0.05 eq) and sodium carbonate (550 mg, 5.19 mmol, 2 eq) were added sequentially to a solution of (4-(methoxycarbonyl)-2-methylphenyl)boronic acid (500 mg, 2.58 mmol, 1 eq) and 2-iodopyridine (600 mg, 2.93 mmol, 1.14 eq) in 1,4-dioxane (4 mL) and water (1 mL) at room temperature under a nitrogen atmosphere. The reaction was carried out at 100 °C for 2 h under a nitrogen atmosphere. After completion of the reaction, the mixture was diluted with water (5 mL) and extracted with EA (3 × 5 mL). The organic phases were combined and washed with saturated sodium chloride solution (2×30 ml), dried over anhydrous sodium sulfate. The resultant was filtered, and concentrated under reduced pressure. The crude product was purified by silica gel column chromatography (EA/PE = 1/3) to afford methyl 3-methyl-4-(pyridin-2-yl)benzoate (375 mg, yield 64%).

LCMS (ESI, m/z): [M+H] ⁺ = 228.0.

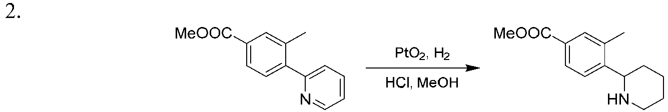

At room temperature, HCl (12 M, 261 µL) and platinum oxide (37 mg, 163 µmol, 0.1 eq) were added to a solution of methyl 3-methyl-4-(pyridin-2-yl)benzoate (370 mg, 1.63 mmol, 1 eq) in methanol (10 mL). The reaction mixture was purged with argon three times and then with hydrogen three times. The reaction was carried at 60 °C under a hydrogen atmosphere (2.0 MPa) overnight. The mixture was filtered to remove the filter cake, and the filtrate was concentrated to dryness by rotary evaporation to give methyl 3-methyl-4-(piperidin-2-yl)benzoate hydrochloride (430 mg).

LCMS (ESI, m/z): [M+H] ⁺ = 234.1.

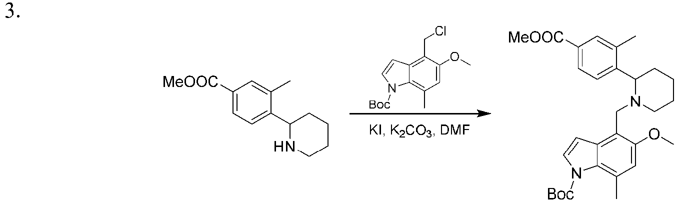

At room temperature under a nitrogen atmosphere, potassium carbonate (750 mg, 5.43 mmol, 3.4 eq) and potassium iodide (300 mg, 1.81 mmol, 1.13 eq) were added, respectively, to a solution of methyl 3-methyl-4-(piperidin-2-yl)benzoate (430 mg, 1.59 mmol, 1 eq) and tert-butyl 4-(chloromethyl)-5-methoxy-7-methyl-1H-indole-1-carboxylate (750 mg, 2.42 mmol, 1.52 eq) in DMF (10 mL). The reaction was carried out at room temperature for 2 h under a nitrogen atmosphere. After completion of the reaction, the mixture was diluted with water (10 mL) and extracted with EA (3 × 10 mL). The organic phases were combined and washed with saturated sodium chloride solution (2×30 ml), dried over anhydrous sodium sulfate. The resultant was filtered, and concentrated under reduced pressure. The crude product was purified by silica gel column chromatography (EA/PE = 1:40 to 1:10) to afford tert-butyl 5-methoxy-4-((2-(4-(methoxycarbonyl)-2-methylphenyl)piperidin-1-yl)methyl)-7-methyl-1H-indole-1-carboxylate (610 mg, yield 75.5%).

LCMS (ESI, m/z): [M+H] ⁺ = 507.3.

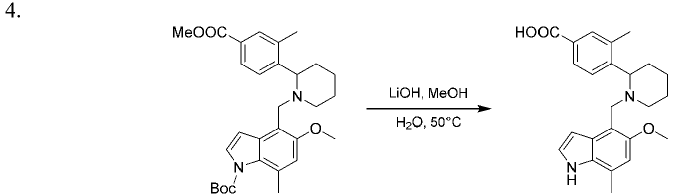

Lithium hydroxide monohydrate (500 mg, 11.9 mmol, 10.1 equiv.) was added to a solution of tert-butyl 5-methoxy-4-((2-(4-(methoxycarbonyl)-2-methylphenyl)piperidin-1-yl)methyl)-7-methyl-1H-indole-1-carboxylate (600 mg, 1.18 mmol, 1.0 eq) in methanol (6 mL) and H₂O (2 mL) at room temperature. The reaction was carried out at 50 °C for 10 h under a nitrogen atmosphere. The reaction mixture was concentrated under reduced pressure. The crude product was purified by preparative HPLC under the following conditions (column: CD20-Waters XBridge BEH C18, 250 × 25 mm, 10 µm; mobile phase: mobile phase A: H₂O (0.1% NH₄HCO₃), mobile phase B: acetonitrile; gradient: 10% B-40% B over 15 min; flow rate: 40 mL/min; wavelength: 254 nm/220 nm; RT = 12.5 min) to afford 4-(1-((5-methoxy-7-methyl-1H-indol-4-yl)methyl)piperidin-2-yl)-3-methylbenzoic acid (100 mg, yield 21.4%).

LCMS (ESI, m/z): [M+H] ⁺ = 393.2.

¹H NMR (400 MHz, DMSO-d6) δ 10.80 (s, 1H), 7.85 (s, 2H), 7.75 (s, 1H), 7.25 (s, 1H), 6.64 (s, 1H), 6.45 (s, 1H), 3.96 (s, 3H), 3.54 (d, J = 12.0 Hz, 1H), 3.39 (m, 2H), 3.15 (d, J = 12.0 Hz, 1H), 2.80 (d, J = 11.2 Hz, 1H), 2.44 (s, 3H), 2.41 (s, 3H), 2.05 - 1.95 (m, 3H), 1.70 - 1.35 (m, 5H).

### Test Examples

The pharmacological activities of the compounds prepared in the Examples of the present invention were evaluated. With reference to the preclinical study data for Comparative Compound D1 (iptacopan), affinity assays, in vitro enzymatic activity assays (CVF-Bb assay, AP-deposition assay, and PNH-like hemolysis assay), and pharmacokinetic studies in rats and mice were conducted, and parallel comparisons with Comparative Compound D1 (iptacopan) were performed.

The preclinical study data for iptacopan demonstrated, through the CVF-Bb assay and the AP-deposition assay, that the drug has potential for treating complement pathway-related nephropathies, such as IgA nephropathy, and hematological diseases by targeting the complement pathway. The inventors evaluated the compounds provided by the present invention using the same experimental items. The pharmacological activities of the compounds of the present invention were demonstrated by comparison with iptacopan.

### Test Example 1: Affinity Assay

The binding affinity of the compounds of the Examples and Comparative Examples to complement factor B was determined to evaluate the binding strength between the compounds and factor B.

### Experimental Reagent:

CFB (Sino Biological), NTA sensors (Sartorius), compound I of Example 1, and Comparative Compounds D1 and D2.

### Experimental Methods:

1) 8 NTA sensors were divided into two groups and pre-wetted in PBST for 10 min or more. 4 sensors were used for immobilization, and the other 4 sensors were used as reference sensors without protein immobilization. PBST was added to the first column of a 96-well sample plate, and CFB at 50 µg/mL was added to the second column. A baseline was set for 60 s, and then loading was performed for 3000 s until the immobilization height reached 4 nm or more. Another baseline was then set for 60 s for equilibration.
2) A new 96-well plate was prepared, and wells were designated as buffer wells and sample wells. PBST + 0.1% DMSO buffer was added to the buffer wells, and compound I, D1, and D2 were added to the sample wells, respectively. The agent concentrations from left to right were 2.47, 7.41, 22.22, 66.67, 200, and 400 nM.
3) After immobilization was completed, a relatively long equilibration step of 10 min or more was first set, and then baseline/association/dissociation cycles were performed. The Assay Definition was set as follows: baseline 60 s, association 120 s, and dissociation 360 s.
4) After the immobilized biosensor cycle was set, a group of reference biosensor cycles was added. The cycle procedure was the same as that used for the immobilized biosensor cycle.
5) After the settings were completed, the positions of the sensors were set in Sensor Assignment. The sensors were divided into two columns, including one group of immobilized sensors and one group of reference sensors, with 4 sensors in each column. After the experiment was checked in Review Experiment, the storage path, file name, temperature, and other parameters were set in Run Experiment, and the run was initiated by clicking GO.
6) The raw data were processed by double subtraction using Octet Data Analysis software, followed by kinetic and steady-state analyses.

### Experimental results:

The binding affinities of the three compounds to complement factor B are shown in Table 1.

**Table 1 Affinity Data**

| Compound | Affinity for factor B |
|---|---|
| D1 | 1.02E⁻⁰⁸M |
| D2 | 1.90E⁻⁰⁸M |
| I | 3.19⁻⁰⁹M |

As shown in Table 1, compound I exhibited higher affinity for complement factor B than Comparative Compounds D1 and D2, indicating that compound I binds more strongly to complement factor B and has potential for treating nephropathies related to abnormal activation of the complement alternative pathway, such as IgA nephropathy, and hematological diseases by targeting CFB.

### Test Example 2: CVF-Bb Assay

Cobra venom factor (CVF) is an anti-complement factor isolated from cobra venom and is closely associated with the complement system. CVF functionally resembles the C3b fragment, a degradation product of C3. Both in vivo and in vitro, CVF can reversibly bind to serum factor B at a 1:1 ratio to form a stable C3 spliceosome CVF-B complex. Upon hydrolytic activation by factor D, the CVF-B complex is cleaved to form CVF-Bb, which has C3 convertase activity. CVF-Bb subsequently cleaves C3 to generate two fragments C3b and C3a, thereby further activating the alternative complement pathway, triggering cascade amplification, and leading to extensive complement activation and depletion.

### Experimental Reagent:

Purified human factor B, purified human factor D (Complement Technology), cobra venom factor, C3 protein (quidel), anti-C3a / C3a des Arg antibody [2991], goat anti-mouse IgG H&L (HRP) (Abcam), QuantaBlu fluorogenic peroxidase substrate kit, Thermo Scientific^{™} StartingBlock^{™} T20 (PBS) 1X (Thermo Fisher), Compound I of Example 1, and comparative compounds D1 and D2.

### Experimental Methods:

1) Factor B, factor D, and CVF were added to the assay wells, mixed, and incubated at 37°C for 3 hours.
2) Compound I, D1, and D2 were separately dissolved in DMSO to prepare 100 mM solutions, which were then diluted 100-fold to prepare 1 mM working solutions. The working solutions were subjected to 3-fold serial dilution to obtain a series of working solutions. For the assay, 1 µL of each working solution was added to the corresponding reaction well to give final compound concentrations of 10000, 3333.33, 1111.11, 370.37, 123.45, 41.15, 13.72, 4.57, and 1.52 nM. After shaking and mixing, the plate was incubated at 37°C for 1 hour.
3) Human complement C3 protein was added to the reaction wells. After mixing, the plate was incubated at 37°C for 2 hours to obtain C3 reaction samples.
4) To each well of a black 96-well immunoassay plate, 97 µL of coating buffer and 3 µL of the reaction sample were added. After mixing, the plate was sealed and incubated overnight at 4°C.
5) The wells were washed three times with 300 µL of washing buffer, followed by addition of 300 µL of StartingBlock blocking buffer. The plate was incubated at room temperature for 15 minutes.
6) The wells were washed three times with 300 µL of washing buffer, and 100 µL of Anti-C3a/C3a des Arg antibody was added. The plate was incubated at 37°C for 1 hour.
7) The wells were washed three times with 300 µL of washing buffer, and 100 µL of goat anti-mouse IgG H&L (HRP) was added. The plate was incubated at 37°C for 30 minutes.
8) The wells were washed three times with 300 µL of washing buffer, and 100 µL of QuantaBlu substrate solution was added. The plate was incubated at room temperature for 20 minutes.
9) Then, 100 µL of QuantaBlu stop solution was added.
10) The absorbance values at 320 nm and 420 nm were measured using a microplate reader.

### Experimental results:

The inhibitory effects of the three compounds on the enzymatic activity of C3 spliceosome are shown in Table 2.

**Table 2. CVF-Bb Enzymatic Activity Data**

| Compound | CVF-Bb assay (IC50) |
|---|---|
| D1 | 16.1nM |
| D2 | 50.9nM |
| I | 11.2nM |

As shown in Table 2, compound I inhibited the enzymatic activity of C3 spliceosome, with an IC₅₀ comparable to that of comparative compound D1. These results indicate that compound I has a significant inhibitory effect on the alternative complement pathway and has potential for treating diseases associated with dysregulation of the alternative complement pathway, including renal diseases such as IgA nephropathy and hematological diseases, by targeting CFB.

### Test Example 3: AP Deposition Assay

In this experiment, lipopolysaccharide (LPS) was used as an activator to initiate the alternative complement pathway. LPS can directly bind to C3b and, in the presence of factors such as factor B and factor D, promote the formation of C3 convertase. C3 convertase cleaves C3 into C3a and C3b. The resulting C3b further associates with the convertase complex to form C5 convertase. C5 convertase then cleaves C5 into C5a and C5b. In the fluid phase, C5b sequentially reacts with C6, C7, C8, and C9 to ultimately form the terminal complement complex (TCC, SC5b-9), thereby exerting a cell-lytic effect.

### Experimental Reagent:

Normal Human Serum (Complement Technology), WIESLAB Complement System Alternative Pathway (Svar Life Science), Compound I of Example 1, and comparative compound D1.

### Experimental Methods:

1) Compound I, D1, and D2 were separately dissolved in DMSO to prepare 1000 mM solutions, which were then diluted 100-fold to prepare 100 µM working solutions. The working solutions were subjected to 3-fold serial dilution to obtain a series of working solutions. For the sample wells, 95 µL of human serum and 5 µL of each test solution were added to each well, giving final compound concentrations of 5000, 1666.67, 555.55, 185.185, 61.728, 20.576, and 6.858 nM. For the control wells, 100 µL of Diluent AP was added as the blank control, NHS positive control was added as the positive control, and NHS negative control was added as the negative control. After mixing, the plate was incubated at 37°C for 1 hour.
2)The plate was washed three times with 300 µL of wash buffer. After the final wash, the plate was tapped dry. Then, 100 µL of C5b-9 conjugate was added, followed by incubation at room temperature for 30 minutes.
3)The plate was washed three times with 300 µL of wash buffer. After the final wash, the plate was tapped dry. Then, 100 µL of substrate solution was added, followed by incubation at room temperature for 30 minutes.
4) The absorbance values at 405 nm were measured using a microplate reader.

### Experimental results:

The AP deposition (enzyme activity) assay data for the two compounds are shown in Table 3.

**Table 3. AP Complement Deposition (Enzyme) Assay Data**

| Compound | AP complement deposition assay |
|---|---|
| D1 | 51.35nM |
| I | 58.5nM |

As shown in Table 3, Compound I exhibited an IC₅₀ value in the AP complement (enzyme activity) deposition assay comparable to that of comparative compound D1. These results indicate that Compound I has potent inhibitory activity against the alternative complement pathway and has potential for the treatment of renal diseases, such as IgA nephropathy, and hematological diseases associated with dysregulation of the alternative complement pathway by targeting CFB.

### Test Example 4: PNH-like Hemolysis Assay

Serum complement is readily activated under acidic conditions (pH 6.4-6.5). Red blood cells from patients with PNH lack CD55 and CD59 and therefore cannot effectively prevent attack by the terminal complement complex, resulting in hemolysis. In contrast, normal red blood cells are resistant to such attack and therefore do not undergo hemolysis. In this assay, anti-CD55 and anti-CD59 antibodies were used to block CD55 and CD59 on the surface of normal red blood cells, thereby mimicking the characteristics of PNH red blood cells. EGTA was used to chelate Ca2+ in serum, thereby blocking the classical and lectin complement pathways while preserving the activity of the alternative complement pathway.

### Experimental Reagent:

C5B-9 antibody, CD55 antibody, EDTA (Thermo), anti-CD59 antibody [MEM-43] (Abcam), PIPES (Macklin), EGTA (Solarbio), whole blood and serum from healthy individuals, Compound I of Example 1, and comparative compounds D1 and D2.

### Experimental Methods:

1) 1 mL of fresh blood was collected from healthy volunteers using heparin anticoagulant tubes, and 15 mL of blood was collected using clot activator tubes.
2) The sample was centrifuged at 2,000 rpm for 5 min at room temperature to remove plasma and other supernatant components. The red blood cells were then washed three times with 10 volumes of sodium chloride solution.
3) The red blood cells were diluted with PBS to 2×10^9 cells/mL.
4) Mouse anti-human CD55 antibody (1 µL, 1 mg/mL) and anti-CD59 monoclonal antibody (3 µL, 1 mg/mL) were added to 100 µL of the red blood cell suspension at 2×10^9 cells/mL. The mixture was incubated at 37 °C for 30 min with shaking.
5) Excessive antibodies were removed by washing the red blood cells three times with PIPES-GVBR solution.
6) The red blood cells were diluted with PIPES-GVBR solution to 1×10^8 cells/mL.
7) Normal human serum (NHS) from the same healthy individual was used as the complement source. Blood was collected into a clot activator tube and centrifuged at 3000 × g for 15 min at 4 °C.
8) EGTA and MgCl2 were added to the serum to block activation of the classical complement pathway (final concentrations: 8 mM and 2.85 mM, respectively); the serum was then acidified to pH 6.4 with 0.25 M HCl to activate the AP system.
9) Serial concentrations of each agent were prepared with the acidified serum and pre-incubated on ice for 10 min; meanwhile, a baseline group (final concentration: 10 mM EDTA group) and a maximum hemolysis group (pure water group) were set up.
10) Drug incubation: 200 µL of acidified serum containing the agent was mixed with 20 µL of red blood cells at 1 × 10^8 cells/mL, followed by incubation with shaking at 37°C for 6 h.
11) Termination of reaction: 200 µL of E-GVBR solution (final EDTA concentration: 15 mM) was added to each tube, followed by centrifugation at 4000 rpm for 5 min at room temperature.
12) Hemolysis detection: 100 µL of the supernatant from each sample was added to a 96-well plate, and the OD was measured at 405 nm.

### Experimental results:

The hemolysis inhibition IC₅₀ values of the three compounds are shown in Table 4.

**Table 4. Serum hemolysis data**

| Compound | Hemolysis Inhibition Rate (%) |
|---|---|
| D1 | 164.6nM |
| D2 | 623.8nM |
| I | 89.84nM |

As shown in Table 4, Compound I exhibited a hemolysis inhibition IC₅₀ value approximately 2-fold better than that of comparative compound D1, suggesting that Compound I may have stronger pharmacological potential for the treatment of paroxysmal nocturnal hemoglobinuria.

### Experimental Example 5. Mouse Pharmacokinetic Study

This experiment was conducted to investigate the bioavailability of Compound I, D1, and D2 in mice after oral gavage administration at 1 mg/kg, and to evaluate their pharmacokinetic characteristics in vivo.

### Experimental Reagent:

BALB/c mice (Vital River), acetonitrile, methanol, isopropanol (chromatographic grade, Merck), formic acid (Macklin), PEG400 (Solarbio), DMSO (Aladdin), Compound I prepared in Example 1, and comparative compounds D1 and D2.

### Experimental Methods:

1) Blood samples of 0.1 mL each were collected from the mice at 5 min, 15 min, 30 min, 1 h, 2 h, 4 h, 6 h, 8 h, 12 h, and 24 h after dosing, and transferred into disposable anticoagulant tubes. The samples were centrifuged at 4000 rpm for 10 min. The plasma was collected and stored at -20°C until analysis.
2) Plasma samples were processed as follows. A 20 µL of plasma sample was placed into a 1.5 mL centrifuge tube, followed by addition of 180 µL of internal standard working solution (10 ng/mL internal standard in acetonitrile). The mixture was vortexed at speed setting 5 for 5 min and then centrifuged at 12,000 rpm for 10 min. The supernatant was collected, transferred into an autosampler vial, and analyzed by LC/MS. Chromatograms were acquired.

### Experimental results:

The bioavailability and pharmacokinetic parameters of the three compounds are shown in Table 5. D1-iv-1 denotes the intravenous administration group, whereas D1-po-1 denotes the oral gavage administration group.

**Table 5. Pharmacokinetic data in mice**

| PK Parameters | T_{1/2} | Tₘₐₓ | C₀/Cₘₐₓ | AUCₗₐₛₜ | AUC_{INF obs} | VZ _{F obs} | Cl _{F obs} | MRTₗₐₛₜ | MRTINF _{obs} | F |
|---|---|---|---|---|---|---|---|---|---|---|
| | h | h | ng/mL | h*ng/mL | h*ng/mL | mL/kg | mL/h/kg | h | h | % |
| D1-iv-1 mg/kg group | 5.77 | - | 2459.79 | 2132.34 | 2216.50 | 3755.47 | 451.16 | 4.38 | 5.44 | - |
| D1-po-1 mg/kg group | 5.22 | 0.500 | 620.61 | 2609.90 | 2708.24 | 2778.22 | 369.24 | 6.35 | 7.26 | 122.19 |
| D2-iv-1 mg/kg group | 1.66 | - | 806.67 | 877.37 | 881.92 | 2719.14 | 1133.89 | 1.85 | 1.91 | - |
| D2-po-1 mg/kg group | 1.73 | 0.50 | 349.90 | 937.46 | 943.54 | 2652.76 | 1059.83 | 2.62 | 2.70 | 106.99 |
| I-iv-1 mg/kg group | 2.61 | - | 1533.65 | 3975.81 | 3982.96 | 944.00 | 251.07 | 3.47 | 3.51 | - |
| I-po-1 mg/kg group | 2.83 | 0.250 | 780.43 | 4134.29 | 4146.24 | 983.41 | 241.18 | 4.54 | 4.61 | 104.10 |

### Experimental Conclusion:

After oral gavage administration at 1 mg/kg in BALB/c mice, Compound I, D1, and D2 all showed high bioavailability. Compared with the Compound I oral gavage group, the systemic exposure (AUC) of the parent drug was lower in the comparative compound D1 and D2 oral gavage groups. In the D2 oral gavage group, the AUCINF _obs was approximately 22.7% of that in the Compound I oral gavage group, and the Cmax was approximately 44.8% of that in the Compound I oral gavage group. D2 also showed faster apparent plasma clearance and a shorter half-life. Taken together, Compound I showed more favorable PK profile than D1 and D2 in BALB/c mice.

### Experimental Example 6. Rat Pharmacokinetic Study

This experiment was conducted to investigate the bioavailability of Compound I, Comparative Example D1 in rats after oral gavage administration at 1 mg/kg, and to evaluate their pharmacokinetic characteristics in vivo.

### Experimental Reagent:

SD rats (Beijing HFK Bioscience), acetonitrile, methanol, and isopropanol (chromatographic grade, Merck), formic acid (Macklin), PEG400 (Solarbio), DMSO (Aladdin), Compound I, and D1.

### Experimental Methods:

1) Blood samples of 0.1 mL each were collected from the mice at 5 min, 15 min, 30 min, 1 h, 2 h, 4 h, 6 h, 8 h, 12 h, and 24 h after dosing, and transferred into disposable anticoagulant tubes. The samples were centrifuged at 4000 rpm for 10 min. The plasma was collected and stored at -20°C until analysis.
2) Plasma samples were processed as follows. A 20 µL of plasma sample was placed into a 1.5 mL centrifuge tube, followed by addition of 180 µL of internal standard working solution (1 ng/mL internal standard in acetonitrile). The mixture was vortexed at speed setting 5 for 10 min and then centrifuged at 12,000 rpm for 10 min. The supernatant was collected, transferred into an autosampler vial, and analyzed by LC- MS/MS. Chromatograms were acquired.

### Experimental results:

The bioavailability and pharmacokinetic parameters of the two compounds are shown in Table 6.

**Table 6. Pharmacokinetic data in rats**

| PK | T1/2 | Tmax | C0/Cmax | AUClast | AUCINF | Vz_F_obs | Cl_F_obs | MRTlast | MRTINF | F |
|---|---|---|---|---|---|---|---|---|---|---|
| Parameters | | | | | _obs | | | | _obs | |
| | h | h | ng/mL | h*ng/mL | h*ng/mL | mL/kg | mL/h/kg | h | h | % |
| D1-po-1 mg/kg group | 4.14 | 0.79 | 379.15 | 2028.57 | 2170.66 | 3016.40 | 494.31 | 5.59 | 7.11 | 102.38 |
| I-po-1 mg/kg group | 3.49 | 0.88 | 545.29 | 2890.13 | 2947.03 | 1719.48 | 359.73 | 4.74 | 5.13 | 136.82 |

### Experimental Conclusion:

Following oral gavage administration of Compound I and Comparative Example D1 to SD rats at 1 mg/kg, both compounds exhibited high oral bioavailability. The systemic exposure of the parent drug (AUC and Cmax) was higher in the Compound I group than that in the Comparative Example D1 group. Taken together, Compound I exhibited a more favorable PK profile than Comparative Example D1 in SD rats.

### Experimental Example 7: LPS-Induced Complement Activation Assay in Mice

Lipopolysaccharide (LPS), an activator of the alternative pathway, can rapidly activate the alternative pathway after administration to mice, thereby triggering a cascade of complement reactions. This results in a marked increase in C3b levels and further amplification of alternative pathway activation. In this experiment, the inhibitory effects of comparative compound D1 and Compound I on the alternative complement pathway were evaluated by measuring changes in plasma C3b levels.

### Experimental Reagent:

C57BL/6J mice (Vital River), LPS (Sigma), C3b Elisa Kit (CUSABIO); Compound I and D1 (Yiling Pharmaceutical).

### Experimental Methods:

After completion of animal quarantine, animals in the model control group and each treatment group were intraperitoneally injected with approximately 0.1 mL/mouse (concentration: 0.5 mg/mL, dose: 2.5 mg/kg) of LPS (prepared in normal saline). Animals in the normal control group were intraperitoneally injected with an equal volume of normal saline. Approximately 1.5 h after model induction, the animals in the treatment groups were administered the corresponding test compounds by oral gavage. The normal control group (blank control group without LPS injection) and the model control group (control group only injected with LPS only) were administered the corresponding volume of vehicle by oral gavage. Approximately 6 h after administration, blood was collected from the mice by eyeball removal (EDTA anticoagulant tubes). The blood samples were centrifuged at 3500 rpm at 4 °C for 10 min, and the plasma was collected and stored at -80 °C for determination of C3b levels.

### Experimental results:

Compared with the model control group, plasma C3b levels were significantly reduced in the D1-120 mg/kg group (P < 0.05), while a decreasing trend of plasma C3b levels was observed in the D1-60 mg/kg group (P > 0.05). Plasma C3b levels were significantly reduced in both the Compound I 60 mg/kg group and the Compound I-120 mg/kg group (P < 0.01). Compared with the D1-60 mg/kg group, the Compound I-60 mg/kg group showed a significant reduction in plasma C3b levels (P < 0.01). Compared with the D1-120 mg/kg group, the Compound I-120 mg/kg group also showed a significant reduction in plasma C3b levels (P < 0.05).

### Experimental Conclusion:

At doses of 60 mg/kg and 120 mg/kg, Compound I significantly inhibited LPS-induced activation of the alternative complement pathway, and its inhibitory effect was superior to that of comparative compound D1.

The C3b content are shown in Table 7.

**Table 7**

| Group | C3b content (ng/ml) |
|---|---|
| Normal control group | 1.60±3.06 |
| Model control group | 113.47±20.20^{##} |
| D1-60mg/kg group | 100.01±14.44 |
| D1-120mg/kg group | 89.89±16.73* |
| Compound I-60mg/kg group | 77.19±8.93**^{△△} |
| Compound I-120mg/kg group | 73.16±5.36**^{$} |

| | |
|---|---|
| Note: ^{#}P < 0.05 and ^{##}P < 0.01 vs. normal control group; *P < 0.05 and **P < 0.01 vs. model control group; ^{△}P < 0.05 and ^{△△}P < 0.01 vs. D1 60 mg/kg group; ^{$}P < 0.05 and ^{$$}P < 0.01 vs. D1 120 mg/kg group. | |

To stabilize the chiral center of Compound I, which is prone to racemization, and thereby improve stability, a series of deuterated analogs were designed based on Compound I, with the aim of maintaining comparable activity while achieving improved stability. Binding affinity assays, in vitro enzymatic/activity assays (including the CVF-Bb assay, AP-deposition assay, and rabbit erythrocyte hemolysis assay), pharmacokinetic studies in dogs, and stability studies were performed on the deuterated analogs and racemic analogs of Compound I, with Compound I included as a parallel comparator, as described below.

### Test Example 8: Rabbit Erythrocyte Hemolysis Assay

A 2% suspension of unsensitized rabbit erythrocytes can activate the alternative complement pathway in serum. In this assay, EGTA was added to rat serum to chelate Ca²⁺, thereby blocking activation of the classical and lectin complement pathways. Because EGTA has relatively low affinity for Mg²⁺, thereby allowing activation of the alternative complement pathway. Alternative pathway complement activity can be evaluated based on the degree of rabbit erythrocyte hemolysis.

### Experimental Reagent:

Rat serum, EGTA (Solarbio), EDTA (Thermo), 2% rabbit erythrocytes (Nanjing SenBeiJia Biological Technology), Compound I, LB220-D01, LB220-D05-1, LB220-D05-2, LB220-D05-3, LB220-D06, LB220-D08, LB220-D08-1, LB220-D08-3, LB220-D08-4, and Compound II (an isomer molecule of Compound I, Yiling Pharmaceutical).

### Experimental Methods:

1) Preparation of erythrocyte suspension: A 10 mL of commercially available 2% rabbit erythrocyte suspension was centrifuged at 2,500 rpm for 5 min at room temperature. The supernatant was discarded, and the erythrocytes were washed twice with 10 mL of normal saline until the supernatant became clear. Then the supernatant was removed, and 10 mL of normal saline was added to the erythrocytes to prepare a 2% rabbit erythrocyte suspension.
2) Drug pretreatment: 10 mL of rat serum was used as the reaction system. EGTA and MgCl₂ were added to the serum to block activation of the classical and lectin complement pathways (final concentrations: 8 mM (62.5x) and 2.85 mM (200X), respectively). Serial concentrations of each test compound were prepared using the treated serum (final drug concentrations: 2500 nM, 1000 nM, 333.33 nM, 111.11 nM, 37.04 nM, and 12.35 nM), and the samples were incubated at room temperature for 10 min; meanwhile, a negative control group (serum containing 10 mM EDTA) and a positive maximum hemolysis group (drug-free serum group) were set up.
3) Drug incubation: For each reaction, 0.2 mL of drug-containing rat serum and negative and positive control (i.e., positive maximum hemolysis group) rat serum were separately mixed with 0.2 mL of 2% erythrocyte suspension (1:1 mixture). After mixing well, the mixtures were immediately placed in a 37 + 0.5°C incubator for shaking incubation (150 rpm), and continuously shaken and incubated for 3 h.
4) Termination of reaction: At the end of the incubation, 200 µL of EDTA-containing normal saline was added to each tube to give a final EDTA concentration of 15 mM. The samples were then centrifuged at 4,000 rpm for 5 min at room temperature.
12) Hemolysis detection: 100 µL of the supernatant from each sample was added to a 96-well plate, and the OD was measured at 405 nm.

### Experimental results:

The IC₅₀ values of LB220-D05-2, LB220-D05-3, LB220-D06, and LB220-D08 for inhibition of rabbit erythrocyte hemolysis were comparable to that of Compound I. In contrast, the other deuterated analogs, including LB220-D05-1, LB220-D01, LB220-D08-1, LB220-D08-3, and LB220-D08-4, as well as Compound II, an isomer of Compound I, did not inhibit rabbit erythrocyte hemolysis.

The rabbit erythrocyte hemolysis are shown in Table 8.

**Table 8**

| Example No. | Compound No. | Rabbit Hemolysis Inhibition Rate(IC₅₀) |
|---|---|---|
| Example 1 | Compound I | 547.6nM |
| Example 3 | LB220-D05-2 | 607.4nM |
| Example 3 | LB220-D05-3 | 537.0nM |
| Example 4 | LB220-D06 | 568.1nM |
| Example 5 | LB220-D08 | 544.2nM |
| Example 2 | LB220-D01 | >1000nM |
| Example 3 | LB220-D05-1 | >1000nM |
| Example 5 | LB220-D08-1 | 1088nM |
| Example 5 | LB220-D08-3 | >1000nM |
| Example 5 | LB220-D08-4 | >1000nM |
| Example 1 | Compound II | >1000nM |

### Test Example 9: Affinity Assay

Based on the results of the in vitro rabbit erythrocyte hemolysis assay, the affinity of Compound I and its deuterated derivatives (the compounds LB220-D05-2 and LB220-D05-3 of Example 3, the compound LB220-D06 of Example 4, and the compound LB220-D08 of Example 5) for complement factor B was determined to evaluate the binding strength of the compounds to factor B.

### Experimental Reagent:

CFB (Sino Biological), NTA sensors (Sartorius), Compound I of Example 1, compounds LB220-D05-2 and LB220-D05-3 of Example 3, compound LB220-D06 of Example 4, and compound LB220-D08 of Example 5 (Yiling Pharmaceutical).

### Experimental Methods:

1) 12 NTA sensors were divided into two groups and pre-wetted in PBST for 10 min or more. 6 sensors were used for immobilization, and the other 6 sensors were used as reference sensors without protein immobilization. PBST was added to the first column of a 96-well sample plate, and CFB at 50 µg/mL was added to the second column. A baseline was set for 60 s, and then loading was performed for 3000 s until the immobilization height reached 4 nm or more. Another baseline was then set for 60 s for equilibration.
2) A new 96-well plate was prepared, and wells were designated as buffer wells and sample wells. PBST + 0.1% DMSO buffer was added to the buffer wells, and compound I, LB220-D05-2, LB220-D05-3, LB220-D06, andLB220-D08 were added to the sample wells, respectively. The agent concentrations from left to right were 2.47, 7.41, 22.22, 66.67, 200, and 400 nM.
3) After immobilization was completed, a relatively long equilibration step of 10 min or more was first set, and then baseline/association/dissociation cycles were performed. The Assay Definition was set as follows: baseline 60 s, association 120 s, and dissociation 360 s.
4) After the immobilized biosensor cycle was set, a group of reference biosensor cycles was added. The cycle procedure was the same as that used for the immobilized biosensor cycle.
5) After the settings were completed, the positions of the sensors were set in Sensor Assignment. The sensors were divided into two columns, including one group of immobilized sensors and one group of reference sensors, with 6 sensors in each column. After the experiment was checked in Review Experiment, the storage path, file name, temperature, and other parameters were set in Run Experiment, and the run was initiated by clicking GO.
6) The raw data were processed by double subtraction using Octet Data Analysis software, followed by kinetic and steady-state analyses.

### Experimental results:

The binding affinities of the five compounds to complement factor B are shown in Table 9.

**Table 9 Affinity Data**

| Compound | Affinity for factor B |
|---|---|
| I | 5.63E⁻⁰⁹M |
| LB220-D05-2 | 1.03E⁻⁰⁸M |
| LB220-D05-3 | 1.53E⁻⁰⁸M |
| LB220-D06 | 4.46E⁻⁰⁹M |
| LB220-D08 | 2.65E⁻⁰⁹M |

As shown in Table 9, compound LB220-D08 of Example 5 exhibited the highest affinity for CFB. The binding strength to CFB was ranked as follows: LB220-D08 > LB220-D06 > I > LB220-D05-2 > LB220-D05-3.

### Test Example 10: CVF-Bb Assay

### Experimental Reagent:

Purified human factor B and purified human factor D (Complement Technology), cobra venom factor and C3 protein (Quidel), Anti-C3a / C3a des Arg protein [2991], goat anti-mouse IgG H&L (HRP) (Abcam), QuantaBlu fluorogenic peroxidase substrate kit, Thermo Scientific^{™} StartingBlock^{™} T20 (PBS) 1X (Thermo Fisher), Compound I of Example 1, compounds LB220-D05-2 and LB220-D05-3 of Example 3, compound LB220-D06 of Example 4, and compound LB220-D08 of Example 5 (Yiling Pharmaceutical).

### Experimental Methods:

1) Factor B, factor D, and CVF were added to the assay wells, mixed, and incubated at 37°C for 3 hours.
2) Compound I, LB220-D05-2, LB220-D05-3, LB220-D06, and LB220-D08 were separately dissolved in DMSO to prepare 100 mM solutions, which were then diluted 100-fold to prepare 1 mM working solutions. The working solutions were subjected to 3-fold serial dilution to obtain a series of working solutions. For the assay, 1 µL of each working solution was added to the corresponding reaction well to give final compound concentrations of 10000, 3333.33, 1111.11, 370.37, 123.45, 41.15, 13.72, 4.57, and 1.52 nM. After shaking and mixing, the plate was incubated at 37°C for 1 hour.
3) Human complement C3 protein was added to the reaction wells. After mixing, the plate was incubated at 37°C for 2 hours to obtain C3 reaction samples.
4) To each well of a black 96-well immunoassay plate, 97 µL of coating buffer and 3 µL of the reaction sample were added. After mixing, the plate was sealed and incubated overnight at 4°C.
5) The wells were washed three times with 300 µL of washing buffer, followed by addition of 300 µL of StartingBlock blocking buffer. The plate was incubated at room temperature for 15 minutes.
6) The wells were washed three times with 300 µL of washing buffer, and 100 µL of Anti-C3a/C3a des Arg antibody was added. The plate was incubated at 37°C for 1 hour.
7) The wells were washed three times with 300 µL of washing buffer, and 100 µL of goat anti-mouse IgG H&L (HRP) was added. The plate was incubated at 37°C for 30 minutes.
8) The wells were washed three times with 300 µL of washing buffer, and 100 µL of QuantaBlu substrate solution was added. The plate was incubated at room temperature for 20 minutes.
9) Then, 100 µL of QuantaBlu stop solution was added.
10) The absorbance values at 320 nm and 420 nm were measured using a microplate reader.

### Experimental results:

The inhibitory effects of the five compounds on of enzymatic activity of C3 spliceosome are shown in Table 10.

**Table 10. CVF-Bb Enzymatic Activity Data**

| Compound | CVF-Bb assay (IC₅₀) |
|---|---|
| I | 12.85nM |
| LB220-D05-2 | 30.57nM |
| LB220-D05-3 | 46.31nM |
| LB220-D06 | 16.01nM |
| LB220-D08 | 12.53nM |

As shown in Table 10, the IC₅₀ of compound LB220-D08 of Example 5 for inhibiting the enzymatic activity of C3 spliceosome was substantially comparable to that of compound I of Example 1. The enzyme inhibitory activity was ranked as follows: LB220-D08 > LB220-D06 > I > LB220-D05-2 > LB220-D05-3.

### Test Example 11: AP Deposition Assay

### Experimental Reagent:

Normal Human Serum (Complement Technology), WIESLAB Complement System Alternative Pathway(Svar Life Science), Compound I of Example 1, compounds LB220-D05-2 and LB220-D05-3 of Example 3, compound LB220-D06 of Example 4, and compound LB220-D08 of Example 5(Yiling Pharmaceutical).

### Experimental Methods:

1) Compound I, LB220-D05-2, LB220-D05-3, LB220-D06, and LB220-D08 were separately dissolved in DMSO to prepare 1000 mM solutions, which were then diluted 100-fold to prepare 100 µM working solutions. The working solutions were subjected to 3-fold serial dilution to obtain a series of working solutions. For the sample wells, 95 µL of human serum and 5 µL of each test solution were added to each well, giving final compound concentrations of 5000, 1666.67, 555.55, 185.185, 61.728, 20.576, and 6.858 nM. For the control wells, 100 µL of Diluent AP was added as the blank control, NHS positive control was added as the positive control, and NHS negative control was added as the negative control. After mixing, the plate was incubated at 37°C for 1 hour.
2)The plate was washed three times with 300 µL of wash buffer. After the final wash, the plate was tapped dry. Then, 100 µL of C5b-9 conjugate was added, followed by incubation at room temperature for 30 minutes.
3)The plate was washed three times with 300 µL of wash buffer. After the final wash, the plate was tapped dry. Then, 100 µL of substrate solution was added, followed by incubation at room temperature for 30 minutes.
4) The absorbance values at 405 nm were measured using a microplate reader.

### Experimental results:

The AP deposition (enzyme activity) assay data for the two compounds are shown in Table 11.

**Table 11. AP Complement Deposition (Enzyme activity) Assay Data**

| Compound | AP complement deposition assay |
|---|---|
| I | 54.85nM |
| LB220-D05-2 | 59.94nM |
| LB220-D05-3 | 66.67 nM |
| LB220-D06 | 55.48nM |
| LB220-D08 | 47.34nM |

As shown in Table 11, compounds LB220-D05-2 and LB220-D05-3 of Example 3, compound LB220-D06 of Example 4, and compound LB220-D08 of Example 5 exhibited IC₅₀ value, substantially comparable to that of compound I of Example 1, in the AP-deposition assay. These results indicate that the above compounds have significant inhibitory activity against the alternative complement pathway and may have potential as CFB-targeting agents for the treatment of diseases associated with dysregulation of the alternative complement pathway, including renal diseases such as IgA nephropathy and hematological diseases.

### Test Example 9. PK study in Beagle Dogs

### Objective of Experiment:

Based on the affinity and in vitro enzymatic activity data, this study was conducted to evaluate the in vivo pharmacokinetic profiles of Compound I, LB220-D06 of Example 4, and LB220-D08 of Example 5 in beagle dogs after each compound was administered separately by oral gavage at 1 mg/kg.

Materials: SD rats (Beijing HFK Bioscience), acetonitrile, methanol, isopropanol (chromatographic grade, Merck), formic acid (Macklin), PEG400 (Solarbio), DMSO (Aladdin), Compound I, LB220-D06, and LB220-D08 (Yiling Pharmaceutical).

### Experimental Methods:

1) Blood samples of 0.1 mL each were collected from the mice at 15 min, 30 min, 1 h, 2 h, 4 h, 6 h, 8 h, 12 h, 24 h, and 48 h after dosing, and transferred into disposable anticoagulant tubes. The samples were centrifuged at 4000 rpm for 10 min. The plasma was collected and stored at -20°C until analysis.
2) Plasma samples were processed as follows. A 20 µL of plasma sample was placed into a 1.5 mL centrifuge tube, followed by addition of 180 µL of internal standard working solution (1 ng/mL internal standard in acetonitrile). The mixture was vortexed at speed setting 5 for 10 min and then centrifuged at 12,000 rpm for 10 min. The supernatant was collected, transferred into an autosampler vial, and analyzed by LC- MS/MS. Chromatograms were acquired.

### Experimental results:

1) After oral gavage administration of Compound I to male beagle dogs at 1 mg/kg, the AUCINF_obs, Tmax, Cmax, and t1/2 were 8568.02 h*ng/mL, 0.42 h, 1473.27 ng/mL, and 5.92 h, respectively.
2) After oral gavage administration of LB220-D06 to male beagle dogs at 1 mg/kg, the AUCINF_obs, Tmax, Cmax, and t1/2 were 8478.75 h*ng/mL, 0.83 h, 1249.67 ng/mL, and 6.46 h, respectively. The relative oral bioavailability was 98.96%.
3) After oral gavage administration of LB220-D08 to male beagle dogs at 1 mg/kg, the AUCINF_obs, Tmax, Cmax, and t1/2 were 9328.75 h*ng/mL, 0.50 h, 1250.78 ng/mL, and 7.03 h, respectively. The relative oral bioavailability was 108.88%.

Following oral gavage administration of LB220-D06 or LB220-D08 at 1 mg/kg in beagle dogs, systemic exposure to the parent compounds ranked as follows: LB220-D08 > Compound I > LB220-D06. However, no marked difference in systemic exposure was observed among the three compounds.

The pharmacokinetic data obtained in dogs are presented in Table 12.

**Table 12**

| Comparison of the Main Plasma PK Parameters of Compound I and LB220-D08 after Intravenous Tail Vein Injection and Oral Gavage Administration at 1 mg/kg in SD Rats | | | | | | | | | | |
|---|---|---|---|---|---|---|---|---|---|---|
| PK Parameters | T1/2 | Tmax | C0/Cmax | AUClast | AUCINF _obs | Vz_F_obs | Cl_F_obs | MRTlast | MRTINF _obs | F |
| | h | h | ng/mL | h*ng/mL | h*ng/mL | mL/kg | mL/h/kg | h | h | % |
| Compound I-po-1 mg/kg group | 5.92 | 0.42 | 1473.27 | 8509.98 | 8568.02 | 1037.81 | 139.62 | 6.15 | 6.41 | - |
| LB220-D06-po-1 mg/kg group | 6.46 | 0.83 | 1249.67 | 8434.01 | 8478.75 | 1115.49 | 121.57 | 7.63 | 7.87 | 98.96 |
| LB220-D08-po-1 mg/kg group | 7.03 | 0.50 | 1250.78 | 9258.45 | 9328.75 | 1131.98 | 111.05 | 7.46 | 7.86 | 108.88 |

### Test example 10: Stability test

Under conditions of 4 N HCl at 50°C for 12 h, LB220-D08 maintained both its chiral purity and chemical purity, with no decrease in either purity observed. In contrast, the Comparative Example (LNP023) and the other deuterated derivatives exhibited varying degrees of decrease in chemical purity and/or chiral purity, as shown in Table 13.

**Table 13**

| | Chiral Purity | Chemical Purity |
|---|---|---|
| Comparative Example (LNP023) | Unchanged | -6.48% |
| Compound I | -0.41% | Unchanged |
| LB220-D01 | -0.49% | Unchanged |
| LB220-D02 | -0.71% | -2.16% |
| LB220-D04 | -0.62% | Unchanged |
| LB220-D05 | Unchanged | -1.22% |
| LB220-D06 | -0.55 | Unchanged |
| LB220-D07 | -0.33% | -4.55% |
| LB220-D08 | **Unchanged** | **Unchanged** |
| LB220-D09 | -0.41% | Unchanged |
| LB220-D10 | Unchanged | -0.29% |
| LB220-D11 | -2.33% | -4.25% |

The foregoing embodiments are provided merely for the purpose of illustrating and explaining the present invention, and are not intended to limit the protection scope of the present invention. Any modifications or improvements made without departing from the principles of the present invention shall also fall within the protection scope of the present invention.

## Claims

1. A compound of Formula A, or a pharmaceutically acceptable form thereof, wherein the pharmaceutically acceptable form is selected from a pharmaceutically acceptable stereoisomer, salt, deuterated derivative, prodrug, polymorph, or solvate:

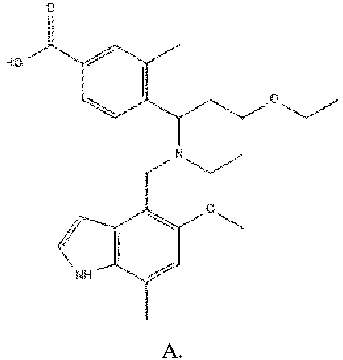

2. A compound of Formula I, or a pharmaceutically acceptable form thereof, wherein the pharmaceutically acceptable form is selected from a pharmaceutically acceptable salt, deuterated derivative, prodrug, polymorph, or solvate:

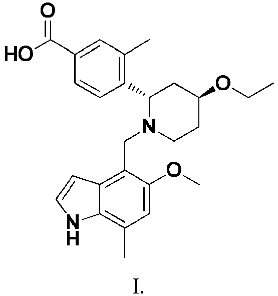

3. The compound or pharmaceutically acceptable form thereof according to claim 2, wherein the deuterated derivative is selected from the following structures:

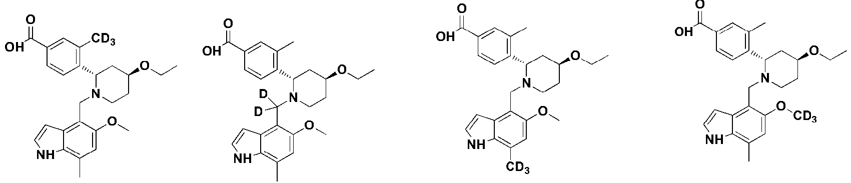

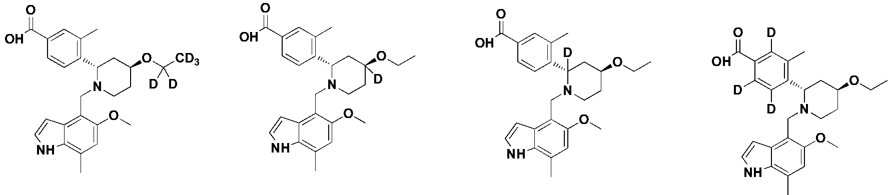

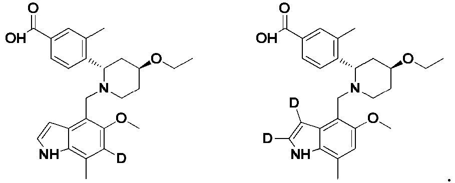

4. A method for preparing the compound according to claim 1, comprising the following steps:
(1) reacting 4-bromo-3-methylbenzonitrile, 4-methoxypyridine, and benzyl chloroformate under the catalyzation action of a Grignard reagent to produce benzyl 2-(4-cyano-2-methylphenyl)-4-oxo-3,4-dihydropyridine-1(2H)-carboxylate;
(2) subjecting the benzyl 2-(4-cyano-2-methylphenyl)-4-oxo-3,4-dihydropyridine-1(2H)-carboxylate to hydrogenation to obtain benzyl 2-(4-cyano-2-methylphenyl)-4-oxopiperidine-1-carboxylate;
(3) reducing the carbonyl group of the benzyl 2-(4-cyano-2-methylphenyl)-4-oxopiperidine-1-carboxylate to obtain benzyl 2-(4-cyano-2-methylphenyl)-4-hydroxypiperidine-1-carboxylate;
(4) reacting benzyl 2-(4-cyano-2-methylphenyl)-4-hydroxypiperidine-1-carboxylate with a silylating reagent in the presence of an acid-binding agent to protect the hydroxy group, thereby obtaining 4-((tert-butyldiphenylsilyl)oxy)-2-(4-cyano-2-methylphenyl)piperidine-1-carboxylate;
(5) removing the hydroxy-protecting group from the 4-((tert-butyldiphenylsilyl)oxy)-2-(4-cyano-2-methylphenyl)piperidine-1-carboxylate to obtain 2-(4-cyano-2-methylphenyl)-4-hydroxypiperidine-1-carboxylate;
(6) subjecting the 2-(4-cyano-2-methylphenyl)-4-hydroxypiperidine-1-carboxylate to a nucleophilic substitution reaction with a halohydrocarbon to obtain 2-(4-cyano-2-methylphenyl)-4-ethoxypiperidine-1-carboxylate;
(7) reacting 2-(4-cyano-2-methylphenyl)-4-ethoxypiperidine-1-carboxylate with an acid and an alkyl alcohol to obtain a 4-(4-ethoxypiperidin-2-yl)-3-methylbenzoate;
(8) reacting the methyl4-(4-ethoxypiperidin-2-yl)-3-methylbenzoate with tert-butyl 4-formyl-5-methoxy-7-methyl-1H-indole-1-carboxylate to obtain tert-butyl 4-(4-ethoxy-2-(4-(alkoxycarbonyl)-2-methylphenyl)piperidin-1-yl)methyl)-5-methoxy-7-methyl-1H-indole-1-carboxylate;
(9) hydrolyzing the tert-butyl 4-(4-ethoxy-2-(4-(methoxycarbonyl)-2-methylphenyl)piperidin-1-yl)methyl)-5-methoxy-7-methyl-1H-indole-1-carboxylate to obtain the compound of Formula A, namely 4-(4-ethoxy-1-((5-methoxy-7-methyl-1H-indol-4-yl)methyl)piperidin-2-yl)-3-methylbenzoic acid.

5. The method for preparing the compound according to claim 4, comprising the following steps:
(1) reacting 4-bromo-3-methylbenzonitrile, 4-methoxypyridine, and benzyl chloroformate under the catalyzation action of a Grignard reagent to produce benzyl 2-(4-cyano-2-methylphenyl)-4-oxo-3,4-dihydropyridine-1(2H)-carboxylate;
(2) subjecting the benzyl 2-(4-cyano-2-methylphenyl)-4-oxo-3,4-dihydropyridine-1(2H)-carboxylate to hydrogenation to obtain benzyl 2-(4-cyano-2-methylphenyl)-4-oxopiperidine-1-carboxylate;
(3) reducing the carbonyl group of the benzyl 2-(4-cyano-2-methylphenyl)-4-oxopiperidine-1-carboxylate to obtain benzyl 2-(4-cyano-2-methylphenyl)-4-hydroxypiperidine-1-carboxylate;
(4) reacting the benzyl 2-(4-cyano-2-methylphenyl)-4-hydroxypiperidine-1-carboxylate with a silylating reagent in the presence of an acid-binding agent to protect the hydroxy group, followed by purification, to obtain trans-4-((tert-butyldiphenylsilyl)oxy)-2-(4-cyano-2-methylphenyl)piperidine-1-carboxylate;
(5) removing the hydroxy-protecting group from the trans-4-((tert-butyldiphenylsilyl)oxy)-2-(4-cyano-2-methylphenyl)piperidine-1-carboxylate to obtain trans-2-(4-cyano-2-methylphenyl)-4-hydroxypiperidine-1-carboxylate;
(6) subjecting the trans-2-(4-cyano-2-methylphenyl)-4-hydroxypiperidine-1-carboxylate to a nucleophilic substitution reaction with a halohydrocarbon to obtain trans-2-(4-cyano-2-methylphenyl)-4-ethoxypiperidine-1-carboxylate;
(7) reacting the trans-2-(4-cyano-2-methylphenyl)-4-ethoxypiperidine-1-carboxylate with an acid and an alkyl alcohol to obtain a trans-4-(4-ethoxypiperidin-2-yl)-3-methylbenzoate;
(8) reacting the trans-4-(4-ethoxypiperldin-2-yl)-3-methylbenzoate with tert-butyl 4-formyl-5-methoxy-7-methyl-1H-indole-1-carboxylate to obtain tert-butyl trans-4-(4-trans-ethoxy-2-(4-(alkoxycarbonyl)-2-methylphenyl)piperidin-1-yl)methyl)-5-methoxy-7-methyl-1H-indole-1-carboxylate;
(9) subjecting the tert-butyl trans-4-(4-transethoxy-2-(4-(alkoxycarbonyl)-2-methylphenyl)piperidin-1-yl)methyl)-5-methoxy-7-methyl-1H-indole-1-carboxylate to SFC purification to obtain tert-butyl 4-(((2S,4S)-4-ethoxy-2-(4-(alkoxycarbonyl)-2-methylphenyl)piperidin-1-yl)methyl)-5-methoxy-7-methyl-1H-indole-1-carboxylate;
(10) hydrolyzing the tert-butyl 4-(((2S,4S)-4-ethoxy-2-(4-(alkoxycarbonyl)-2-methylphenyl)piperidin-1-yl)methyl)-5-methoxy-7-methyl-1H-indole-1-carboxylate to obtain the compound of Formula I, namely 4-((2S,4S)-4-ethoxy-1-((5-methoxy-7-methyl-1H-indol-4-yl)methyl)piperidin-2-yl)-3-methylbenzoic acid.

6. The method according to claim 4 or 5, wherein the Grignard reagent in step (1) is isopropylmagnesium chloride; and/or
the silylating reagent in step (4) is tert-butyldiphenylsilyl chloride, and the acid-binding agent is imidazole; and/or
the halohydrocarbon in step (6) is ethyl iodide; and/or
the acid in step (7) is sulfuric acid, and the alkyl alcohol is methanol.

7. A pharmaceutical composition, comprising the compound or pharmaceutically acceptable form thereof according to any one of claims 1, 2, or 3, and a pharmaceutically acceptable carrier, excipient, and/or one or more additional therapeutic agents.

8. Use of the compound or pharmaceutically acceptable form thereof according to any one of claims 1, 2, or 3, in the manufacture of a medicament for treating a disease or disorder mediated by dysregulation of the alternative complement pathway.

9. Use of the compound or pharmaceutically acceptable form thereof according to any one of claims 1, 2, or 3, in the manufacture of a medicament for treating a disease or disorder mediated by complement factor B.

10. Use of the compound or pharmaceutically acceptable form thereof according to any one of claims 1, 2, or 3, in the manufacture of a medicament for treating a hematological, autoimmune, inflammatory, and/or neurodegenerative disease or disorder.

11. Use of the compound or pharmaceutically acceptable form thereof according to any one of claims 1, 2, or 3, in the manufacture of a medicament for treating a disease mediated by dysregulation of the alternative complement pathway, wherein the disease is selected from Paroxysmal nocturnal hemoglobinuria, Immunoglobulin A nephropathy, C3 glomerulopathy, Atypical hemolytic uremic syndrome, Age-related macular degeneration, ANCA-associated vasculitis, Systemic lupus erythematosus, Immune thrombocytopenia, and Cold agglutinin disease.
